# EUROPEAN PATENT APPLICATION

(11) **EP 4 711 470 A1**
(43) Date of publication of application: **18.03.2026**
(21) Application number: 24839727.5
(22) Date of filing: 08.07.2024
(51) Int. Cl.: C12Q 1/32, G01N 33/533

(54) **METHOD AND KIT FOR EVALUATING ENZYME ACTIVITY**

(30) Priority: 11.07.2023 JP 2023113583
(71) Applicant: The University of Tokyo, Bunkyo-ku, Tokyo 113-8654 (JP)
(72) Inventor: KOMATSU, Toru, Tokyo 113-8654 (JP); URANO, Yasuteru, Tokyo 113-8654 (JP); KAGAMI, Yu, Tokyo 113-8654 (JP); SAKAMOTO, Shingo, Tokyo 113-8654 (JP)
(74) Representative: Vossius & Partner Patentanwälte Rechtsanwälte mbB
(86) International application number: PCT/JP2024/024651
(87) International publication number: WO 2025/013837

(57) **Abstract**

Disclosed is a method for evaluating the oxidation activity of a dehydrogenase, the method comprising causing the dehydrogenase and DT-diaphorase to catalyze an oxidation-reduction reaction in an aqueous solution containing one molecule of the dehydrogenase, a substrate of the dehydrogenase, a nucleoside diphosphate derivative, the DT-diaphorase, and a fluorescent probe, and measuring the fluorescence after the reaction.

## Description

### Technical Field

The present disclosure relates to a method and a kit for evaluating enzyme activity.

### Background Art

An enzyme is a biopolymer composed mainly of a protein, which acts as a catalyst for a chemical reaction carried out in a living body. In a living body, there exist more than several thousand kinds of enzymes, and homeostasis of a living body is maintained by metabolic reactions performed by these. It is also known that an abnormality in these functions is related to many diseases. An activity of an enzyme in a living body is regulated by various factors, such as not only an amino acid sequence thereof but also a post-translational modification, or allosteric regulation by a metabolite. Therefore, in evaluating an enzyme derived from a biological sample, in addition to merely evaluating an expression level thereof and a change at a gene level, evaluating an activity of the enzyme is extremely effective.

In a living body, there exist multiple subtypes that, while targeting the same substrate, have different activities depending on a tissue and the like from which they are derived. Among these subtypes, there also exist those having an extremely small expression level, and it is inferred that, among such subtypes having a small expression level, there possibly exist those whose activity is enhanced only in a specific disease such as cancer. However, according to a general enzyme activity evaluation method such as an evaluation using a standard substrate, an enzyme activity of a subtype having a small expression level is buried and cannot be detected as a significant difference, because an activity of all enzymes present in a sample is evaluated as a sum (bulk). In evaluating an activity of such a subtype having a small expression level, an evaluation method for evaluating an activity of an enzyme at the single-molecule level is useful (Non Patent Literature 1). In addition, a microchamber that makes it possible to perform such a single-molecule analysis has been reported (Patent Literature 1).

A dehydrogenase is an oxidase that oxidizes a substrate by using, as a coenzyme, an oxidized nucleoside diphosphate derivative such as an oxidized nicotinamide adenine dinucleotide derivative or an oxidized flavin adenine dinucleotide derivative. There are many dehydrogenases among enzymes that constitute a metabolic pathway such as a glycolytic pathway. In addition, there also exist many dehydrogenases whose association with a disease including cancer has been reported.

The present inventors have developed a coupled assay system for detecting an activity of a D-lactate dehydrogenase, which is a kind of dehydrogenase (Non Patent Literature 2).

### Citation List

### Patent Literature

Patent Literature 1: US 9797837 (B2)

### Non Patent Literature

Non Patent Literature 1: Yannick Rondelez et al., "Micro fabricated arrays of femtoliter chambers allow single molecule enzymology", Nat. Biotech. 23(3), 361-365 (2005).
Non Patent Literature 2: Kouichi Yanagi et al., "Development of pathway-oriented screening to identify compounds to control 2-methylglyoxal metabolism in tumor cells", Commun.Chem. 6, 68 (2023).
Non Patent Literature 3: Narae Shin et al., "Development of an Azoreductase-based Reporter System with Synthetic Fluorogenic Substrates", ACS Chem. Biol. 12(2):558-563 (2017).
Non Patent Literature 4: Chengli Jia et al., "A fast-responsive fluorescent turn-on probe for nitroreductase imaging in living cells", RSC Advances 11(15):8516-8520 (2021).
Non Patent Literature 5: Kouichi Yanagi et al., "Establishment of live-cell-based coupled assay system for identification of compounds to modulate metabolic activities of cells", Cell Reports., 36(1), 109311 (2021).

### Summary of Invention

### Technical Problem

To date, there has been no known example in which an oxidative activity of a dehydrogenase has been detected at the single-molecule level. Also, to date, there has been no known example in which a coupled assay for detecting an activity of an enzyme to be evaluated has been performed under a condition in which the enzyme to be evaluated exists only at the single-molecule level.

Also, according to a conventional evaluation method, in order to directly evaluate an oxidative activity of a dehydrogenase by using a fluorescent probe, it is necessary to design and develop, for each dehydrogenase, a fluorescent probe that becomes a substrate of the dehydrogenase and that increases fluorescence intensity by an enzymatic reaction. Furthermore, in single molecule activity detection, in order to detect and distinguish signals of multiple subtypes from a pattern of fluorescence, it is necessary to develop multiple fluorescent probes that become a substrate of a certain dehydrogenase. However, since there are many dehydrogenases having high substrate specificity, it is extremely difficult to develop multiple fluorescent probes that serve as substrates for those dehydrogenases.

An object of the present disclosure is to provide a method for evaluating an oxidative activity of a dehydrogenase. Also, an object of the present disclosure is to provide a method for evaluating an enzyme activity at the single-molecule level.

### Solution to Problem

The present inventors have found that an oxidative activity can be evaluated at the single-molecule level of the dehydrogenase by a coupled assay in an aqueous solution containing a single molecule of a dehydrogenase, a substrate of the dehydrogenase, a nucleoside diphosphate derivative, a DT-diaphorase, and a fluorescent probe. Furthermore, the present inventors have also found that, according to such a method, even when one kind of fluorescent probe is used, different patterns of fluorescence are obtained from the same sample by using substrates of various dehydrogenases and/or nucleoside diphosphate derivatives, and thereby the present disclosure has been completed.

The present inventors have also found that an oxidative activity can be evaluated at the single-molecule level of the metabolic enzyme by a coupled assay in an aqueous solution containing a single molecule of a metabolic enzyme, a substrate of the metabolic enzyme, a dehydrogenase, a nucleoside diphosphate derivative, a DT-diaphorase, and a fluorescent probe.

The present disclosure relates to, for example, the following [1] to [11].
[1] A method for evaluating an oxidative activity of a dehydrogenase, comprising the following steps (A) to (C):
   Step (A): a step of preparing an aqueous solution containing a single molecule of the dehydrogenase, a substrate of the dehydrogenase, a nucleoside diphosphate derivative, a DT-diaphorase, and a fluorescent probe;
   Step (B): a step of causing the dehydrogenase and the DT-diaphorase to catalyze a redox reaction in the aqueous solution prepared in the step (A); and
   Step (C): a step of measuring fluorescence of the aqueous solution resulting from the step (B);
   wherein the dehydrogenase is capable of using an oxidized form of the nucleoside diphosphate derivative as a coenzyme,
   wherein the DT-diaphorase is capable of using a reduced form of the nucleoside diphosphate derivative as a coenzyme,
   and wherein the fluorescent probe is a compound whose fluorescence intensity increases upon reduction by the DT-diaphorase.
[2] The evaluation method according to [1], wherein the step (A) is a step of filling an aqueous solution containing the dehydrogenase, the substrate of the dehydrogenase, the nucleoside diphosphate derivative, the DT-diaphorase, and the fluorescent probe into a plurality of accommodating portions of a microchamber array having a hydrophobic surface, the step comprising the following steps (i) and (ii) in this order:
   (i) from an opening of each of the accommodating portions, flowing the aqueous solution containing the dehydrogenase, the substrate of the dehydrogenase, the nucleoside diphosphate derivative, the DT-diaphorase, and the fluorescent probe into each of the plurality of accommodating portions so that a number-average number of molecules of the dehydrogenase to be filled in each of the plurality of accommodating portions is 0.0002 to 0.5; and
   (ii) closing the opening with a hydrophobic solvent.
[3] The evaluation method according to [1] or [2], wherein the fluorescent probe has at least one substituent selected from the group consisting of -CO₂H, -PO₃H₂, and -SO₃H.
[4] The evaluation method according to any one of [1] to [3], wherein the nucleoside diphosphate derivative in an oxidized form is at least one compound selected from the group consisting of compounds represented by the following formula (I) or (II): 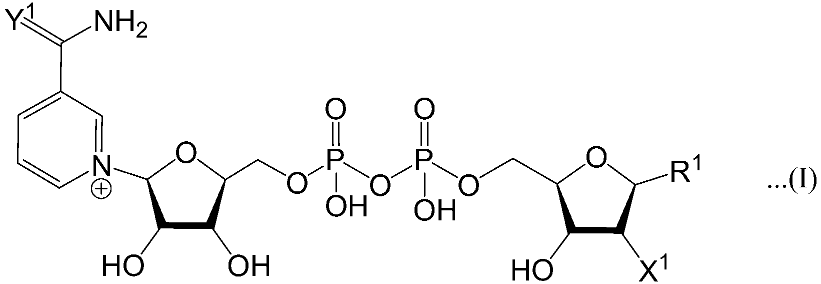 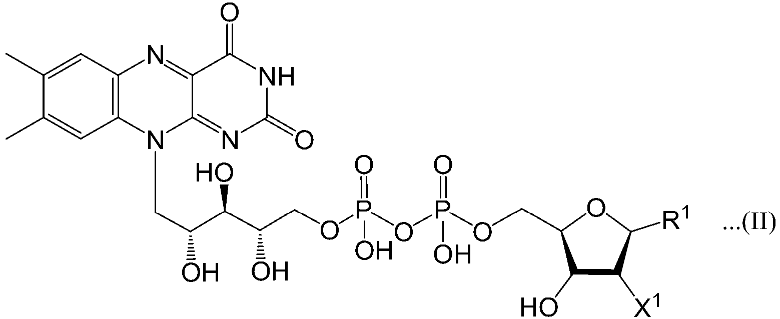 , wherein R¹ is a monovalent group, X¹ is -H, -OH, or -PO₃H₂, and Y₁ is O or S.
[5] The evaluation method according to any one of [1] to [4], wherein the DT-diaphorase is an NADH:quinone oxidoreductase, an azoreductase, or a nitroreductase.
[6] The evaluation method according to any one of [1] to [5], wherein the dehydrogenase is a dehydrogenase contained in at least one selected from the group consisting of plasma, serum, urine, saliva, tears, cerebrospinal fluid, tissue homogenate, cell homogenate, and extracts thereof.
[7] A method for evaluating an oxidative activity of a dehydrogenase, comprising the following steps (A-1) to (C-1) and steps (A-2) to (C-2):
   Step (A-1): a step of preparing an aqueous solution containing a single molecule of the dehydrogenase, a substrate of the dehydrogenase, a first nucleoside diphosphate derivative, a DT-diaphorase, and a fluorescent probe;
   Step (B-1): a step of causing the dehydrogenase and the DT-diaphorase to catalyze a redox reaction in the aqueous solution prepared in the step (A-1);
   Step (C-1): a step of measuring fluorescence of the aqueous solution resulting from the step (B-1);
   Step (A-2): a step of preparing an aqueous solution containing a single molecule of the dehydrogenase, the substrate of the dehydrogenase, a second nucleoside diphosphate derivative, the DT-diaphorase, and the fluorescent probe;
   Step (B-2): a step of causing the dehydrogenase and the DT-diaphorase to catalyze a redox reaction in the aqueous solution prepared in the step (A-2); and
   Step (C-2): a step of measuring fluorescence of the aqueous solution resulting from the step (B-2);
   wherein the first nucleoside diphosphate derivative is different from the second nucleoside diphosphate derivative,
   wherein the dehydrogenase is capable of using an oxidized form of the first nucleoside diphosphate derivative and an oxidized form of the second nucleoside diphosphate derivative as a coenzyme,
   wherein the DT-diaphorase is capable of using a reduced form of the first nucleoside diphosphate derivative and a reduced form of the second nucleoside diphosphate derivative as a coenzyme,
   and wherein the fluorescent probe is a compound whose fluorescence intensity increases upon reduction by the DT-diaphorase.
[8] A method for evaluating an oxidative activity of a dehydrogenase, comprising the following steps (A-3) to (C-3) and steps (A-4) to (C-4):
   Step (A-3): a step of preparing an aqueous solution containing a single molecule of a first dehydrogenase, a substrate of the first dehydrogenase, a nucleoside diphosphate derivative, a DT-diaphorase, and a fluorescent probe;
   Step (B-3): a step of causing the first dehydrogenase and the DT-diaphorase to catalyze a redox reaction in the aqueous solution prepared in the step (A-3);
   Step (C-3): a step of measuring fluorescence of the aqueous solution resulting from the step (B-3);
   Step (A-4): a step of preparing an aqueous solution containing a single molecule of a second dehydrogenase, a substrate of the second dehydrogenase, the nucleoside diphosphate derivative, the DT-diaphorase, and the fluorescent probe;
   Step (B-4): a step of causing the second dehydrogenase and the DT-diaphorase to catalyze a redox reaction in the aqueous solution prepared in the step (A-4); and
   Step (C-4): a step of measuring fluorescence of the aqueous solution resulting from the step (B-4);
   wherein the substrate of the first dehydrogenase is different from the substrate of the second dehydrogenase,
   wherein the first dehydrogenase and the second dehydrogenase are capable of using an oxidized form of the nucleoside diphosphate derivative as a coenzyme,
   wherein the DT-diaphorase is capable of using a reduced form of the nucleoside diphosphate derivative as a coenzyme,
   and wherein the fluorescent probe is a compound whose fluorescence intensity increases upon reduction by the DT-diaphorase.
[9] A kit for evaluating an oxidative activity of a dehydrogenase, comprising a substrate of the dehydrogenase, a nucleoside diphosphate derivative, a DT-diaphorase, and a fluorescent probe,
   wherein the dehydrogenase is capable of using an oxidized form of the nucleoside diphosphate derivative as a coenzyme,
   wherein the DT-diaphorase is capable of using a reduced form of the nucleoside diphosphate derivative as a coenzyme,
   and wherein the fluorescent probe is a compound whose fluorescence intensity increases upon reduction by the DT-diaphorase.
[10] A method for evaluating a metabolic activity of a metabolic enzyme, comprising the following steps (A) to (C):
   Step (A): a step of preparing an aqueous solution containing a single molecule of the metabolic enzyme, a substrate of the metabolic enzyme, a dehydrogenase, a nucleoside diphosphate derivative, a DT-diaphorase, and a fluorescent probe;
   Step (B): a step of causing the metabolic enzyme, the dehydrogenase, and the DT-diaphorase to catalyze an enzymatic reaction in the aqueous solution prepared in the step (A); and
   Step (C): a step of measuring fluorescence of the aqueous solution resulting from the step (B);
   wherein the dehydrogenase is a dehydrogenase that uses, as a substrate, a metabolite resulting from the metabolic enzyme metabolizing the substrate of the metabolic enzyme,
   wherein the dehydrogenase is capable of using an oxidized form of the nucleoside diphosphate derivative as a coenzyme,
   wherein the DT-diaphorase is capable of using a reduced form of the nucleoside diphosphate derivative as a coenzyme,
   and wherein the fluorescent probe is a compound whose fluorescence intensity increases upon reduction by the DT-diaphorase.
[11] A kit for evaluating a metabolic activity of a metabolic enzyme, comprising a substrate of the metabolic enzyme, a dehydrogenase, a nucleoside diphosphate derivative, a DT-diaphorase, and a fluorescent probe,
   wherein the dehydrogenase is a dehydrogenase that uses, as a substrate, a metabolite resulting from the metabolic enzyme metabolizing the substrate of the metabolic enzyme,
   wherein the dehydrogenase is capable of using an oxidized form of the nucleoside diphosphate derivative as a coenzyme
   wherein the DT-diaphorase is capable of using a reduced form of the nucleoside diphosphate derivative as a coenzyme,
   and wherein the fluorescent probe is a compound whose fluorescence intensity increases upon reduction by the DT-diaphorase.

### Advantageous Effects of Invention

According to the present disclosure, a method for evaluating an oxidative activity of a dehydrogenase is provided. According to the present disclosure, it a method for evaluating an enzyme activity at the single-molecule level is also provided.

According to one embodiment of the present disclosure, an oxidative activity of a dehydrogenase can be evaluated (detected) at the single-molecule level of an enzyme.

According to one embodiment of the present disclosure, even when one kind of fluorescent probe is used, it is possible to detect (evaluate) oxidative activities of multiple types of dehydrogenases by using substrates of various dehydrogenases.

According to one embodiment of the present disclosure, even when one kind of fluorescent probe is used, it is possible to detect (evaluate) oxidative activities of multiple types of dehydrogenases by using various nucleoside diphosphate derivatives.

According to one embodiment of the present disclosure, by utilizing that an oxidative activity changes in a dehydrogenase derived from a sample that meets a specific condition, it is possible to evaluate whether the sample meets the specific condition by detecting an enzyme activity of a dehydrogenase in the sample. In this case, according to one embodiment of the present disclosure, even when one kind of fluorescent probe is used, by using substrates of various dehydrogenases and various nucleoside diphosphate derivatives, it is possible to find a coupled assay system suitable for evaluating such a sample that meets such a specific condition.

### Brief Description of Drawings

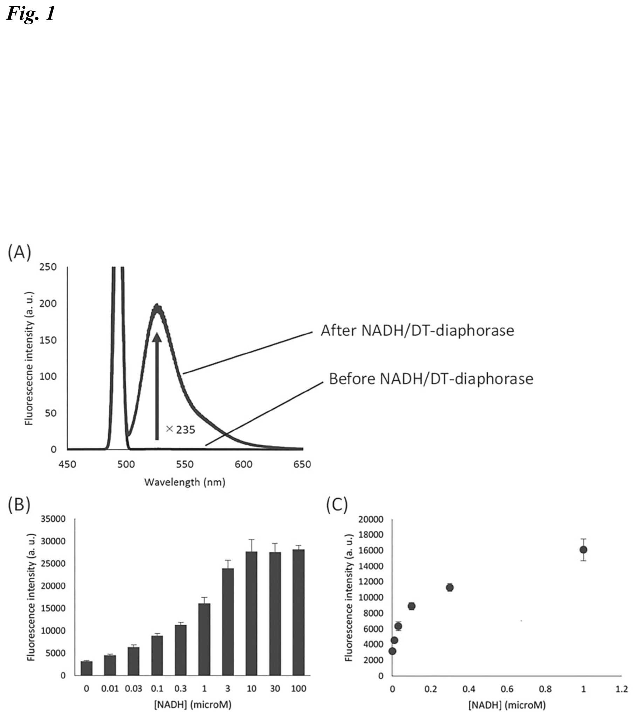

(A) of FIG. 1 is a diagram showing absorption spectra of Q-sHMRG before and after a reduction reaction by NADH:quinone oxidoreductase in Reference Example 1. (B) of FIG. 1 is a diagram showing fluorescence intensity derived from Q-sHMRG when a reduction reaction by NADH:quinone oxidoreductase was performed in the presence of various concentrations of NADH in Reference Example 1. (C) of FIG. 1 is a diagram showing fluorescence intensity derived from Q-sHMRG when a reduction reaction by NADH:quinone oxidoreductase was performed in the presence of various concentrations of NADH in Reference Example 1.
FIG. 2 is a diagram showing fluorescence images and line plots of fluorescence intensities when an evaluation method according to one embodiment of the present disclosure was performed using plasma derived from healthy subjects as a sample in Example 1. Q-sHMRG, Q-HMRG, or resazurin was used as a fluorescent probe, lactate was used as a substrate of a dehydrogenase, and NAD⁺ was used as a nucleoside diphosphate derivative, respectively.
FIG. 3 is a diagram showing a fluorescence image when an evaluation method according to one embodiment of the present disclosure was performed using plasma derived from healthy subjects as a sample in Example 2. Substrates of various dehydrogenases were used as substrates of a dehydrogenase and NAD⁺ was used as a nucleoside diphosphate derivative, respectively.
FIG. 4 is a diagram showing a fluorescence image when an evaluation method according to one embodiment of the present disclosure was performed using plasma derived from healthy subjects as a sample in Example 3. Lactate, glucose-6-phosphate, isocitrate, or 6-phosphogluconate was used as a substrate of a dehydrogenase and NAD⁺, NADP⁺, Thio-NAD⁺, or NDG⁺ was used as a nucleoside diphosphate derivative, respectively.
FIG. 5 is a diagram showing a fluorescence image when an evaluation method according to one embodiment of the present disclosure was performed using plasma derived from healthy subjects or from patients with pancreatic cancer as a sample in Example 4. Glucose-6-phosphate or 6-phosphogluconate was used as a substrate of a dehydrogenase and NAD⁺, NADP⁺, or Thio-NAD⁺ was used as a nucleoside diphosphate derivative, respectively.
FIG. 6 is a diagram showing results of evaluating activities of metabolic enzymes contained in plasma derived from patients with pancreatic cancer in Example 5.
FIG. 7 is a diagram showing fluorescence images obtained under a condition in which glucose-6-phosphate was added, using samples derived from mice that were not subjected to TAA treatment (control group) or mice that were subjected to TAA treatment (TAA group) in Example 6.
FIG. 8 is a diagram showing fluorescence images obtained under a condition in which lactate was added, using samples derived from mice that were not subjected to TAA treatment (control group) or mice that were subjected to TAA treatment (TAA group) in Example 6.
FIG. 9 is a histogram of fluorescence images acquired under the same conditions as in FIG. 7, showing distributions of fluorescence intensity for each well in which fluorescence was detected in Example 6.
FIG. 10 is a diagram of fluorescence images acquired under the same conditions as in FIG. 7 and FIG. 8, showing a proportion of wells in which fluorescence was detected (lambda) in Example 6.
FIG. 11 is a diagram showing fluorescence images obtained when CSF samples derived from PCNSL patients (PCNSL group) and subjects in whom PCNSL regressed (Regression group) were used in Example 7.
FIG. 12 is a diagram showing results of counting numbers of wells in which fluorescence was observed in fluorescence images obtained under the same conditions as in FIG. 11 when CSF samples derived from PCNSL patients (PCNSL group), subjects in whom PCNSL regressed (Regression group), patients in whom PCNSL recurred (Recurrence group), and subjects in whom PCNSL remitted (Remission group) were used in Example 7.
FIG. 13 is a diagram showing rates of increase in fluorescence (/min) in the presence of substrates of various metabolic enzymes when a plasma sample was added to give 1000-fold or 100-fold dilution in Comparative Example 1.
FIG. 14 is a diagram showing a fluorescence image of a device 20 hours after addition of plasma in Example 8.

### Description of Embodiments

Hereinafter, embodiments for carrying out the present disclosure will be described in detail; however, the present disclosure is not limited to the following embodiments.

In the present disclosure, unless otherwise specified, amino acid means an L-form amino acid. For example, in the present disclosure, descriptions "alanine" and "L-alanine" mean L-form alanine, and a description "D-alanine" means D-form alanine.

### <First aspect>

A first aspect of the present disclosure is a method for evaluating an oxidative activity of a dehydrogenase, comprising the following steps (A) to (C):
Step (A): a preparation step of preparing an aqueous solution containing a single molecule of the dehydrogenase, a substrate of the dehydrogenase, a nucleoside diphosphate derivative, a DT-diaphorase, and a fluorescent probe;
Step (B): a reaction step of causing the dehydrogenase and the DT-diaphorase to catalyze a redox reaction in the aqueous solution prepared in Step (A); and
Step (C): a fluorescence measurement step of measuring fluorescence of the aqueous solution resulting from Step (B);
wherein the dehydrogenase is capable of using an oxidized form of the nucleoside diphosphate derivative as a coenzyme, wherein the DT-diaphorase is capable of using a reduced form of the nucleoside diphosphate derivative as a coenzyme, and wherein the fluorescent probe is a compound whose fluorescence intensity increases upon reduction by the DT-diaphorase. That is, the first aspect of the present disclosure is a method of performing a coupled assay shown in the following reaction scheme at the single-molecule level of the dehydrogenase. In the present disclosure, a coupled assay means an enzyme assay performed by a combination of multiple enzymatic reactions. In the coupled assay shown in the following reaction scheme, by making an enzymatic reaction by the dehydrogenase the rate-limiting step of an entire coupled assay system, in other words, by making a reduced-form nucleoside diphosphate derivative generated by the enzymatic reaction of the dehydrogenase promptly consumed for a reduction reaction of the fluorescent probe by the DT-diaphorase, it becomes possible to evaluate an oxidative activity of the dehydrogenase by using fluorescence of a reduction product of the enzymatic reaction by the DT-diaphorase as an index.

Substrate of
Dehydrogenase
Oxidized form of
Nucleoside diphosphate
derivative
Reduced product
（Strongly fluorescent）
Dehydrogenase
DT-diaphorase
Oxidized product
Reduced form of
Nucleoside diphosphate
derivative
Fluorescent probe
（Weakly fluorescent）

A dehydrogenase is an oxidase (oxidoreductase) that oxidizes a substrate by using, as a coenzyme, an oxidized nucleoside diphosphate derivative such as an oxidized nicotinamide adenine dinucleotide derivative or an oxidized flavin adenine dinucleotide derivative. Dehydrogenases are abundant among enzymes constituting a metabolic pathway such as a glycolytic pathway, and there also exist many dehydrogenases whose association with diseases including cancer has been reported.

In the present disclosure, a dehydrogenase is not particularly limited as long as it is an oxidase (oxidoreductase) that can oxidize a substrate by using an oxidized nucleoside diphosphate derivative as a coenzyme. In a preferred embodiment of the present disclosure, a dehydrogenase is an oxidase (oxidoreductase) that can oxidize a substrate by using an oxidized nicotinamide adenine dinucleotide derivative or an oxidized flavin adenine dinucleotide derivative as a coenzyme. In a preferred embodiment of the present disclosure, a dehydrogenase may be an enzyme classified as EC 1.1.1, EC 1.2.1, EC 1.3.1, EC 1.4.1, EC 1.4.4, EC 1.5.1, EC 1.6 (for example EC 1.6.1), EC 1.7.1, EC 1.8.1, EC 1.10.1, EC 1.12.1, EC 1.14.12, EC 1.14.13, EC 1.14.21, EC 1.17.1, EC 1.18.1, EC 1.19.1, EC 1.20.1, EC 1.21.1, or EC 1.23.1, in EC7 of an EC classification established in 2019 by the Nomenclature Committee of the International Union of Biochemistry and Molecular Biology (NC-IUBMB). In a more preferred embodiment, a dehydrogenase may be an enzyme classified, in EC7, as EC 1.1.1, EC 1.2.1, EC 1.3.1, EC 1.4.1, EC 1.4.4, EC 1.5.1, EC 1.6 (for example EC 1.6.1), EC 1.7.1, EC 1.8.1, EC 1.10.1, EC 1.12.1, EC 1.14.12, EC 1.14.13, EC 1.14.21, EC 1.18.1, EC 1.19.1, or EC 1.21.1. In an even more preferred embodiment, a dehydrogenase may be an enzyme classified, in EC7, as EC 1.1.1 or EC 1.2.1. Examples of a dehydrogenase according to one embodiment of the present disclosure include lactate dehydrogenase, isocitrate dehydrogenase, alpha-ketoglutarate dehydrogenase, succinate dehydrogenase, malate dehydrogenase, glucose-6-phosphate dehydrogenase, 6-phosphogluconate dehydrogenase, alcohol dehydrogenase, aldehyde dehydrogenase, Cytochrome P450, glutathione reductase, and HMG-CoA reductase, and the like.

In one embodiment of the present disclosure, a dehydrogenase may be a dehydrogenase contained in at least one selected from the group consisting of plasma, serum, urine, saliva, tears, cerebrospinal fluid, tissue homogenate, cell homogenate, and extracts thereof. In a preferred embodiment, a dehydrogenase may be a dehydrogenase contained in at least one selected from the group consisting of plasma, serum, urine, saliva, tears, and cerebrospinal fluid, and extracts thereof, and in an even more preferred embodiment, a dehydrogenase may be a dehydrogenase contained in at least one selected from the group consisting of plasma, serum, tears, and cerebrospinal fluid, and extracts thereof. When a sample from which the dehydrogenase is derived is evaluated through evaluation of an oxidative activity of the dehydrogenase by the evaluation method according to one embodiment of the present disclosure, the sample may be at least one selected from the group consisting of plasma, serum, urine, saliva, tears, cerebrospinal fluid, tissue homogenate, cell homogenate, and extracts thereof.

### <Substrate of Dehydrogenase >

A substrate of a dehydrogenase according to the present disclosure is not particularly limited as long as it becomes a substrate of a dehydrogenase and is oxidized by the dehydrogenase. A substrate of a dehydrogenase according to the present disclosure may be a natural substrate of a dehydrogenase, and may also be an artificial substrate such as a derivative of such a natural substrate.

As a substrate of a dehydrogenase according to the present disclosure, for example, glycine, serine, D-serine, glutamic acid, lysine, leucine, glutathione, glucose-6-phosphate, lactate, D-lactate, isocitrate, malate, inosinic acid, 6-phosphogluconate, succinic semialdehyde, terephthalaldehydic acid, and HMG-CoA (hydroxymethylglutaryl CoA) can be used.

When Step (B) (a reaction step) of the first aspect of the present disclosure starts, a concentration of a substrate of the dehydrogenase is not particularly limited as long as it is a concentration at which fluorescence can be detected in a subsequent Step (C) (a fluorescence measurement step), but for example may be 10 µM to 1 M, and may also be 30 µM to 100 mM, 100 µM to 30 mM, or 300 µM to 10 mM.

### <Nucleoside diphosphate derivative>

A nucleoside diphosphate derivative according to the present disclosure is a nucleoside diphosphate derivative the oxidized form of which the dehydrogenase is capable of using as a coenzyme, and the reduced form of which the DT-diaphorase is capable of using as a coenzyme. In one embodiment of the present disclosure, a nucleoside diphosphate derivative may be a nicotinamide adenine dinucleotide derivative or a flavin adenine dinucleotide derivative, and, for example, may be at least one compound selected from the group consisting of compounds represented by the following formula (I) or (II): 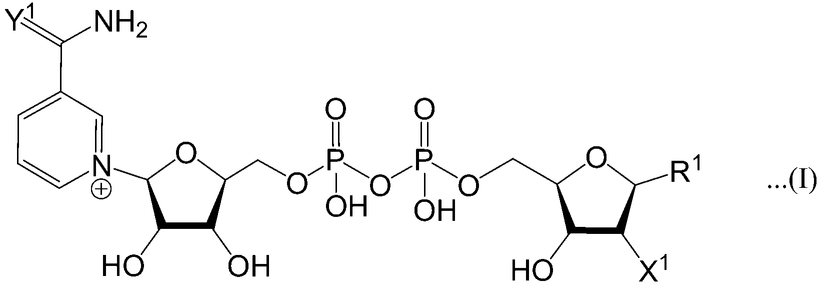 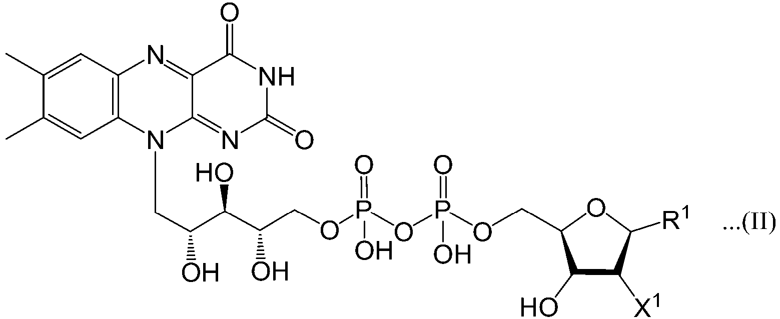 , wherein R¹ is a monovalent group, X¹ is -H, -OH, or -PO₃H₂, and Y¹ is O or S. In a reduced form, the compounds represented by the formula (I) and the formula (II) are represented by the following formula (I') and formula (II'), respectively. 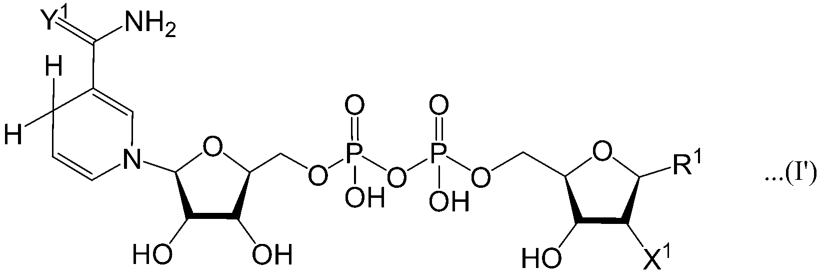 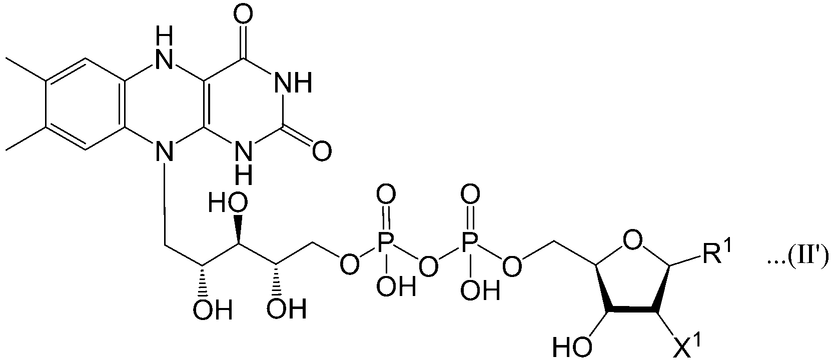 Also, a nucleoside diphosphate derivative according to the present disclosure may be in a form of a salt, a solvate, or a solvate of a salt, and, for example, may be a sodium salt, a hydrochloride, or a hydrate.

A nucleoside diphosphate derivative according to the present disclosure may be a compound represented by the formula (I) or (II) above, and, in a more preferred embodiment and in a most preferred embodiment, may be a compound represented by the formula (I) above. In the formula (I) or (II) above, X¹ is -H, -OH, or -PO₃H₂, and, for example, may be -OH or -PO₃H₂. In the formula (I) or (II) above, in a preferred embodiment, R¹ may be a monovalent group selected from a group consisting of substituents represented by the following structural formula. 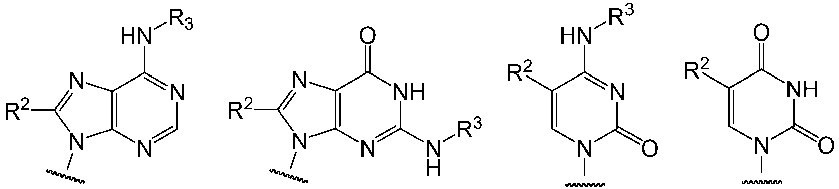 In the above formula, R² is -H, an alkyl group, an alkylamino group, or an (aminoalkyl)amino group. In the above formula, R³ is a monovalent group, and is preferably H, an alkyl group, an acyl group, a benzyl group, or a furfuryl group, more preferably -H, a benzyl group, a furfuryl group, or a group represented by -C(=O)R^{3a}, and even more preferably -H or a group represented by -C(=O)R^{3a}, wherein R^{3a} represents an alkyl group. In these cases, an alkyl contained in the alkyl group, the alkylamino group, and the (aminoalkyl)amino group is preferably a C₁-C₆ alkyl, more preferably a C₁-C₄ alkyl, and even more preferably methyl, ethyl, or n-propyl. In a more preferred embodiment, R¹ may be a monovalent group selected from a group consisting of substituents represented by the following structural formula. 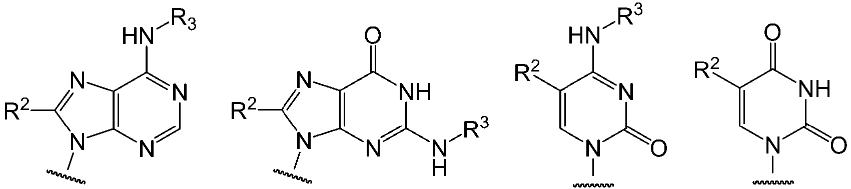 In the above formula, R² is -H or a methyl group, and R³ is a monovalent group. In a most preferred embodiment, R¹ may be at least one represented by the following structural formula. 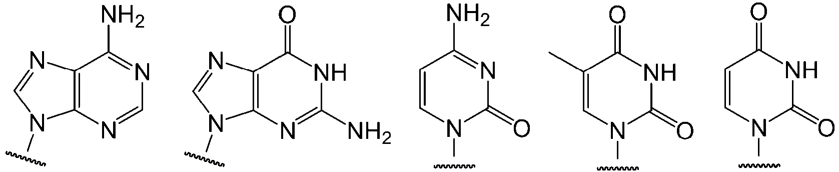 That is, in a most preferred embodiment, R¹ may be a group obtained by removing one hydrogen atom at a position corresponding to a phosphate modification site in monophosphates of adenine (A), guanine (G), cytosine (C), thymine (T), or uracil (U), which are bases present in a living body (AMP, GMP, CMP, TMP, or UMP).

When Step (B) (a reaction step) of the first aspect of the present disclosure starts, a concentration of a nucleoside diphosphate derivative is not particularly limited as long as it is a concentration at which fluorescence can be detected in a subsequent Step (C) (a fluorescence measurement step), but for example may be 1 µM to 10 mM, and may also be 3 µM to 3 mM, 10 µM to 1 mM, 30 µM to 800 µM, or 100 µM to 500 µM.

### <DT-diaphorase>

A DT-diaphorase according to the present disclosure is an enzyme that is capable of reducing the fluorescent probe according to the first aspect of the present disclosure by using a reduced form of the nucleoside diphosphate derivative according to the first aspect of the present disclosure as a coenzyme, and is not particularly limited as long as it is such an enzyme. As the DT-diaphorase according to the present disclosure, for example, an NADH:quinone oxidoreductase, an azoreductase, or a nitroreductase may be used, and, in a preferred embodiment, an NADH:quinone oxidoreductase or an azoreductase may be used, and, in a more preferred embodiment, an NADH:quinone oxidoreductase may be used. An origin of the NADH:quinone oxidoreductase according to the present disclosure is not particularly limited, and may be, for example, human-derived (NQO1, NAD(P)H:Quinone Oxidoreductase 1) or microorganism-derived (DAD-311, DT-Diaphorase-311), and, in a preferred embodiment, may be human-derived (NQO1).

When Step (B) (a reaction step) of the first aspect of the present disclosure starts, a concentration of a DT-diaphorase is not particularly limited as long as it is a concentration at which fluorescence can be detected in a subsequent Step (C) (a fluorescence measurement step), but for example may be 0.1 µg/mL to 100 µg/mL, and may also be 0.5 µg/mL to 60 µg/mL or 1.0 µg/mL to 30 µg/mL.

When Step (B) (a reaction step) of the first aspect of the present disclosure starts, a reaction rate constant k [/s] of an enzymatic reaction by the DT-diaphorase in an aqueous solution, which is defined as a product of a number of molecules of the DT-diaphorase present in the aqueous solution and a turnover number kcat (/s) of the DT-diaphorase, is not particularly limited as long as it is a value at which fluorescence can be detected in a subsequent Step (C) (a fluorescence measurement step), but for example may be 0.001 or more, may be 0.003 or more, and is preferably 0.01 or more, 0.03 or more, or 0.1 or more. When the reaction rate constant k of the enzymatic reaction by the DT-diaphorase in the aqueous solution in Step (B) (the reaction step) is equal to or greater than the lower limit described above, a rate-limiting step in an entire coupled assay tends to be an enzymatic reaction by the dehydrogenase, and therefore it becomes easier to evaluate an oxidative activity of the dehydrogenase.

### <Fluorescent probe>

A fluorescent probe according to the first aspect of the present disclosure is a compound whose fluorescence intensity increases upon reduction by the DT-diaphorase according to the first aspect of the present disclosure. A fluorescent probe according to the first aspect of the present disclosure can be designed or selected in accordance with substrate properties of the DT-diaphorase according to the first aspect of the present disclosure. A fluorescence intensity of a compound (a reduction product) obtained by the fluorescent probe being reduced by the DT-diaphorase may be, for example, 2 times or more, 3 times or more, 4 times or more, 5 times or more, 7 times or more, 10 times or more, 15 times or more, 20 times or more, 30 times or more, 50 times or more, 70 times or more, or 100 times or more than that of the fluorescent probe, and, in a preferred embodiment, may be 5 times or more. These fluorescence intensities may be values measured, for example, in phosphate-buffered saline (PBS) at pH 7.4.

A compound (a reduction product) obtained by a fluorescent probe according to the first aspect of the present disclosure being reduced by a DT-diaphorase has a mathematical product of a molar extinction coefficient [L/mol·cm] at a maximum absorption wavelength and a fluorescence quantum yield that may be, for example, 2000 or more, 5000 or more, 10000 or more, 15000 or more, 20000 or more, 30000 or more, 40000 or more, or 50000 or more, and, in a preferred embodiment, may be 10000 or more. For example, sHMRG shown by the following structural formula has a molar extinction coefficient of 80000 and a fluorescence quantum yield of 0.92, and a mathematical product thereof is 74000.

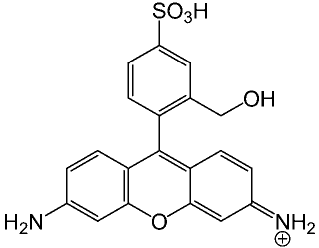

Also, for example, 7-amino-4-methylcoumarin has a molar extinction coefficient of 20000 and a fluorescence quantum yield of 0.42, and a mathematical product thereof is 8400. These fluorescence characteristics may be values measured, for example, in phosphate-buffered saline (PBS) at pH 7.4.

A maximum absorption wavelength of a compound (a reduction product) obtained by a fluorescent probe according to the first aspect of the present disclosure being reduced by a DT-diaphorase may be, for example, 300 nm or more, 320 nm or more, 340 nm or more, 360 nm or more, 380 nm or more, 400 nm or more, 410 nm or more, 420 nm or more, 430 nm or more, 440 nm or more, 450 nm or more, 460 nm or more, 470 nm or more, 475 nm or more, 480 nm or more, 485 nm or more, 490 nm or more, 495 nm or more, or 500 nm or more, and, in a preferred embodiment, may be 450 nm or more. For example, a maximum absorption wavelength of sHMRG is 498 nm and a maximum fluorescence wavelength is 517 nm, and a maximum absorption wavelength of 7-amino-4-methylcoumarin is 350 nm and a maximum fluorescence wavelength is 440 nm. These fluorescence characteristics may be values measured, for example, in phosphate-buffered saline (PBS) at pH 7.4. When a compound obtained by a fluorescent probe being reduced by a DT-diaphorase has fluorescence characteristics as described above, fluorescence becomes easier to measure in Step (C), and evaluation of an activity of a dehydrogenase becomes easier.

In a preferred embodiment of the first aspect of the present disclosure, a fluorescent probe has at least one substituent selected from the group consisting of -CO₂H, -PO₃H₂, and -SO₃H, and in a more preferred embodiment, may have at least one substituent represented by -SO₃H. When a fluorescent probe has these substituents, solubility of the fluorescent probe in an aqueous solvent in Step (B) is improved, and therefore fluorescence becomes easier to detect in a subsequent Step (C) through securing a concentration and suppressing precipitation.

In a preferred embodiment of the first aspect of the present disclosure, a fluorescent probe according to the first aspect of the present disclosure and/or a compound obtained by the fluorescent probe being reduced by a DT-diaphorase may have high water solubility, and a fluorescent probe according to the first aspect of the present disclosure and a compound obtained by the fluorescent probe being reduced by a DT-diaphorase may have high water solubility. High water solubility may mean, for example, that, when 1 µM of a compound is added to a two-phase system of octanol and phosphate-buffered saline (PBS) at pH 7.4 at a volume ratio of 1:1, a proportion of the compound distributed in a PBS layer is, for example, 50% or more, 60% or more, 70% or more, 80% or more, 85% or more, 90% or more, 95% or more, 97% or more, 98% or more, or 99% or more.

In a preferred embodiment of the first aspect of the present disclosure, a fluorescent probe according to the first aspect of the present disclosure may have high reactivity with a DT-diaphorase. High reactivity with a DT-diaphorase may mean, for example, that, in a buffer close to physiological conditions (for example, Dulbecco's phosphate-buffered saline (DPBS) at pH 7.4) containing 1 U/mL of a DT-diaphorase and a predetermined concentration of an oxidized nucleoside diphosphate derivative (for example, NAD⁺ or NADP⁺), a fluorescence intensity when 1 µM of a fluorescent probe is added and then incubated at 37°C for 1 hour is significantly larger (for example, a p-value in Student's t-test is less than 0.05 or less than 0.01) than a fluorescence intensity when incubated under the same conditions except that the oxidized nucleoside diphosphate derivative is not contained, and the predetermined concentration may be, for example, 0.03 mM, 0.1 mM, 0.3 mM, or 1 mM, and, preferably, may be 0.1 mM.

In the present disclosure, a fluorescent probe may be in a form of a salt, a solvate, or a solvate of a salt. As a counter ion in such a salt or a solvate of the salt, it is not particularly limited, but may be, for example, a sodium ion, a potassium ion, an ammonium ion, a triethylammonium ion, an N,N-diisopropylethylammonium ion, a tetra-n-butylammonium ion, a chloride ion, an acetate ion, or a trifluoroacetate ion. Also, as a solvent that solvates in such a solvate or a solvate of a salt, it is not particularly limited, but may be, for example, water, acetonitrile, dimethyl sulfoxide (DMSO), methanol, acetone, dimethylformamide (DMF), or triethylamine.

A fluorescent probe according to the first aspect of the present disclosure may be represented, for example, by the following formula (III):

X-NH-Y ... (III)

or formula (III').

X-N=Y' ... (III')

In this case, in the formula (III) and the formula (III'), X represents a fluorescent moiety or a partial structure thereof, Y and Y' represent a leaving group removed by an enzymatic reaction of a DT-diaphorase according to the first aspect of the present disclosure, and a fluorescence intensity of a compound represented by a structural formula X-NH₂ is greater than a fluorescence intensity of a compound represented by a structural formula X-NH-Y and a fluorescence intensity of a compound represented by a structural formula X-N=Y'. Hereinafter, a compound represented by a structural formula X-NH-Y and a compound represented by a structural formula X-N=Y' are also described as "a pre-elimination compound," and a compound represented by a structural formula X-NH₂ (a reduction product of a reduction reaction by a DT-diaphorase) is also described as "a post-elimination compound." In this case, regarding fluorescence intensities of pre- and post-elimination compounds, for example, a fluorescence intensity of a post-elimination compound at a pH approximately equal (for example, within ±1.0, within ±0.5, within ±0.2, within ±0.1, within ±0.05, or within ±0.01) to a pH of an aqueous solution in Step (B) is greater than a fluorescence intensity of a pre-elimination compound under the same conditions. The fluorescence intensity in these cases may be a fluorescence intensity at a maximum fluorescence wavelength of the post-elimination compound when excited with excitation light at a wavelength within a wavelength range of a wavelength region of excitation light irradiated in Step (C), for example. Also, a fluorescence intensity of a post-elimination compound may be, for example, 2 times or more, 3 times or more, 4 times or more, 5 times or more, 7 times or more, 10 times or more, 15 times or more, 20 times or more, 30 times or more, 50 times or more, 70 times or more, or 100 times or more than that of a pre-elimination compound, and, in a preferred embodiment, may be 5 times or more. Also, a product of a molar extinction coefficient [L/mol·cm] at a maximum absorption wavelength and a fluorescence quantum yield of a post-elimination compound may be, for example, 2000 or more, 5000 or more, 10000 or more, 15000 or more, 20000 or more, 30000 or more, 40000 or more, or 50000 or more, and, in a preferred embodiment, may be 10000 or more. Also, a maximum absorption wavelength of a post-elimination compound may be, for example, 300 nm or more, 320 nm or more, 340 nm or more, 360 nm or more, 380 nm or more, 400 nm or more, 410 nm or more, 420 nm or more, 430 nm or more, 440 nm or more, 450 nm or more, 460 nm or more, 470 nm or more, or 480 nm or more, and, in a preferred embodiment, may be 420 nm or more. A maximum fluorescence wavelength of a post-elimination compound may be, for example, 400 nm or more, 420 nm or more, 430 nm or more, 440 nm or more, 450 nm or more, 460 nm or more, 470 nm or more, 475 nm or more, 480 nm or more, 485 nm or more, 490 nm or more, 495 nm or more, or 500 nm or more, and, in a preferred embodiment, may be 450 nm or more. When a post-elimination compound has fluorescence characteristics as described above, fluorescence becomes easier to measure in Step (C), and evaluation of an activity of a dehydrogenase becomes easier.

When a DT-diaphorase according to the first aspect of the present disclosure is an NADH:quinone oxidoreductase, a fluorescent probe according to the first aspect of the present disclosure may be, for example, a compound represented by the following formula (IV): 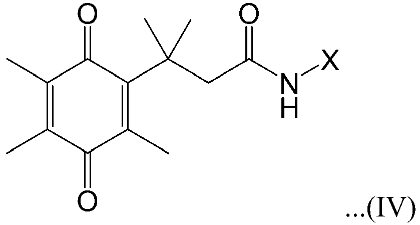 , wherein X is the same as X in the formula (III), or a compound represented by the following formula (V): 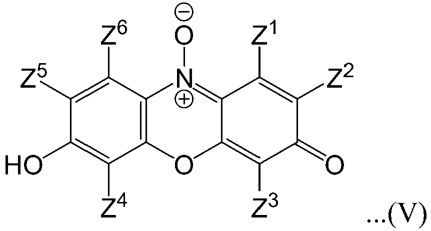 , wherein Z¹ to Z⁶ each independently represent a monovalent group, preferably each independently represent -H, -CO₂H, -PO₃H₂, -SO₃H, or an alkyl group, and more preferably each independently represent -H, - CO₂H, -PO₃H₂, or -SO₃H, and, preferably may be a compound represented by the formula (IV).

A compound represented by the formula (IV) produces X-NH₂, which is a molecule having large fluorescence intensity, by an enzymatic reaction (a reduction reaction) by an NADH:quinone oxidoreductase shown below.

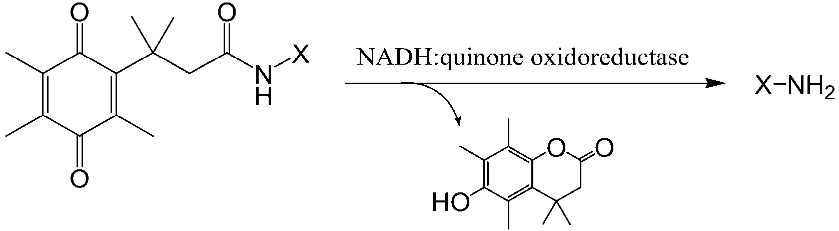

An Example of a compound represented by the formula (V) is resazurin. Resazurin produces resorufin, which is a molecule having large fluorescence intensity, by an enzymatic reaction (a reduction reaction) by an NADH:quinone oxidoreductase shown below.

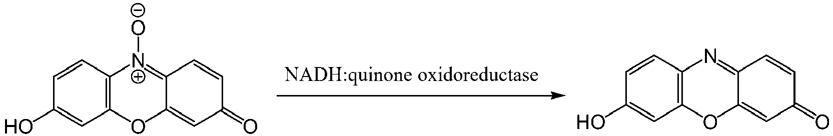

When a DT-diaphorase according to the first aspect of the present disclosure is an azoreductase, a fluorescent probe according to the first aspect of the present disclosure may be, for example, a compound represented by the following formula (VI): 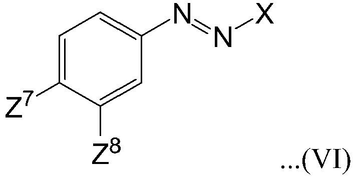 , wherein X is the same as X in the formula (III), and Z⁷ and Z⁸ each independently represent a monovalent group. As such a fluorescent probe, for example, those described in Non Patent Literature 3 can be used. Also, among fluorescent probes, particularly a structure of a portion other than X can be designed or selected with reference to Non Patent Literature 3, for example. A compound represented by the formula (VI) produces X-NH₂, which is a molecule having large fluorescence intensity, by an enzymatic reaction (a reduction reaction) by an azoreductase.

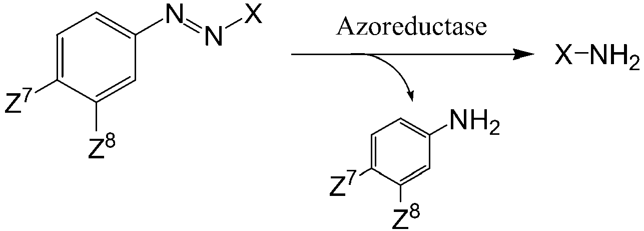

Also, a fluorescent probe according to the first aspect of the present disclosure may be a fluorescent probe in which a self-cleavable group whose terminal amino group is in a precursor form depending on a site eliminated or reduced by an enzymatic reaction of a DT-diaphorase is bound to a fluorescent moiety via an ether bond or a carbamate bond. A self-cleavable group is, for example, p-aminobenzyl, o-aminobenzyl, or aminomethyl, may be p-aminobenzyl or o-aminobenzyl in one embodiment, and, in a preferred embodiment, may be p-aminobenzyl. A precursor of a terminal amino group of a self-cleavable group may be, for example, an azo group, a nitro group, or an amide group.

A fluorescent probe having a self-cleavable group whose terminal amino group is in a precursor form as described above may be, for example, a probe in which X-* in the formula (IV) or the formula (VI) is replaced with X-O-L-* or X-NH-C(=O)-O-L-* (L is a self-cleavable group from which the terminal amino group was removed, and * represents a bond). For example, a fluorescent probe in which X-* in the formula (VI) is replaced with X-O-L-*, and a self-cleavable group is p-aminobenzyl, causes elimination of p-aminobenzyl, which is a self-cleavable group generated as a partial structure, by an azo group being reduced to an amino group by an enzymatic reaction (a reduction reaction) by an azoreductase as shown below, and produces X-OH, which is a molecule having large fluorescence intensity. In the formula, Z⁷, Z⁸, and X are the same as in the formula (VI).

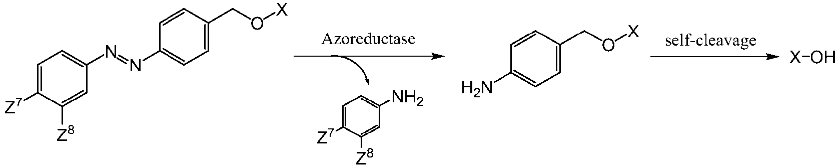

A fluorescent probe having a self-cleavable group whose terminal amino group is in a precursor form as described above may be, for example, a fluorescent probe in which a group represented by X-O-* or X-NH-C(=O)-O-* is bound to p-nitrobenzyl or o-nitrobenzyl. For example, a fluorescent probe in which a group represented by X-O-* is bound to p-nitrobenzyl causes elimination of p-aminobenzyl, which is a self-cleavable group generated as a partial structure, by a nitro group being reduced to an amino group by an enzymatic reaction (a reduction reaction) by a nitroreductase as shown below, and produces X-OH, which is a molecule having large fluorescence intensity. Also, in a fluorescent probe in which a group represented by X-NH-C(=O)-O-* is bound to a nitrobenzyl, in addition to elimination of aminobenzyl, decarboxylation occurs, and X-NH₂, which is a molecule having large fluorescence intensity, is produced.

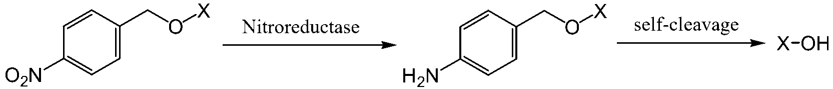

When a DT-diaphorase according to the first aspect of the present disclosure is a nitroreductase, a fluorescent probe according to the first aspect of the present disclosure may be, for example, a compound represented by the following formula (VII): 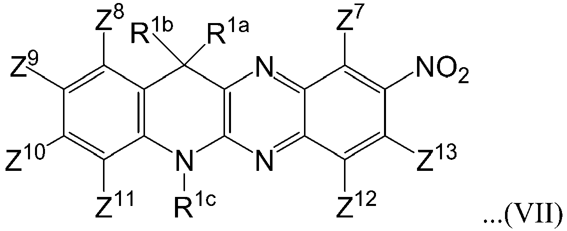 , wherein Z⁷ to Z¹³ each independently represent a monovalent group, Z⁷, Z⁸, and Z¹⁰ to Z¹³ preferably each independently represent -H, -CO₂H, -PO₃H₂, -SO₃H, or an alkyl group, more preferably each independently represent -H, -CO₂H, -PO₃H₂, or -SO₃H, Z⁹ preferably represents -CF₃, and R^{1a} to R^{1c} each independently represent a monovalent group, preferably represent -H or an alkyl group, more preferably represent an alkyl group, even more preferably represent a methyl group, an ethyl group, or an n-propyl group, and yet more preferably represent a methyl group. Example of such a fluorescent probe is NTR-NO₂ shown below. NTR-NO₂ produces NTR-NH₂, which is a molecule having large fluorescence intensity, by an enzymatic reaction (a reduction reaction) by a nitroreductase (Non Patent Literature 4).

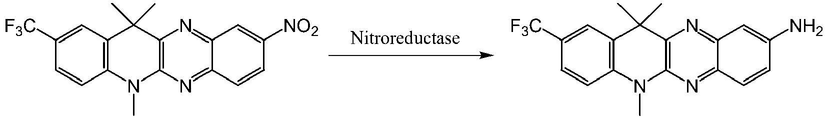

In a preferred embodiment of the first aspect of the present disclosure, X in the formulas (III), (III'), (IV), and (VI) has at least one water-soluble substituent selected from the group consisting of -CO₂H, -PO₃H₂, and -SO₃H, and may be one selected from the groups represented by the following formulas (VIII) to (XIII): 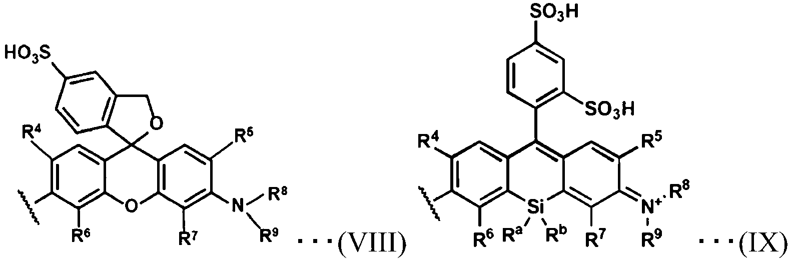 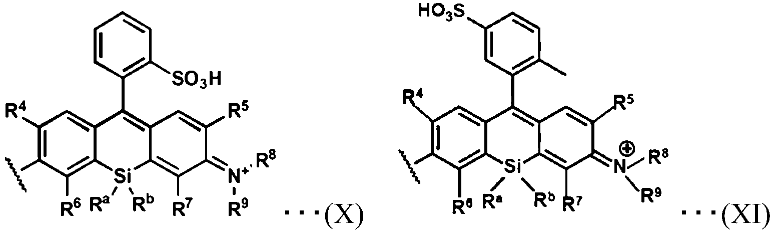 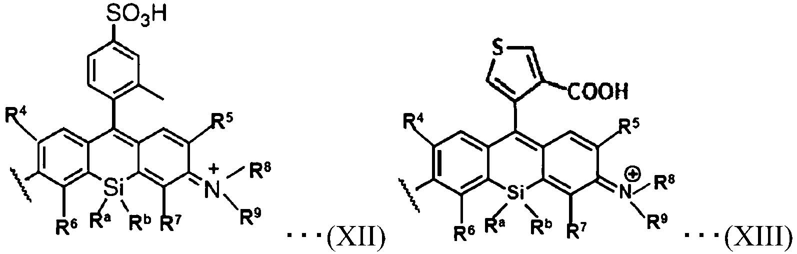 , wherein
R⁴ and R⁵ each independently are a hydrogen atom, a substituted or unsubstituted alkyl group having 1 to 6 carbon atoms, a halogen atom, the water-soluble substituent, or an alkyl group having 1 to 6 carbon atoms having the water-soluble substituent, preferably are a hydrogen atom, a methyl group, an ethyl group, or a halogen atom, more preferably are a hydrogen atom or a halogen atom, and even more preferably are a hydrogen atom;
R⁶ and R⁷ each independently are a hydrogen atom, a substituted or unsubstituted alkyl group having 1 to 6 carbon atoms, a halogen atom, the water-soluble substituent, or an alkyl group having 1 to 6 carbon atoms having the water-soluble substituent, preferably are a hydrogen atom, a methyl group, an ethyl group, or a halogen atom, more preferably are a hydrogen atom or a halogen atom, and even more preferably are a hydrogen atom;
R⁸ and R⁹ each independently are a hydrogen atom, a substituted or unsubstituted alkyl group having 1 to 6 carbon atoms, a substituted or unsubstituted alkenyl group having 1 to 6 carbon atoms, an alkyl group having 1 to 6 carbon atoms having the water-soluble substituent, or an alkenyl group having 1 to 6 carbon atoms having the water-soluble substituent, preferably are a hydrogen atom or an unsubstituted alkyl group having 1 to 6 carbon atoms, more preferably are a hydrogen atom, a methyl group, or an ethyl group, and even more preferably are a hydrogen atom,
wherein R⁸ and R⁹ may together form a heterocyclyl having a substituent and having 4 to 7 members including a nitrogen atom to which R⁸ and R⁹ are bonded, R⁸ or R⁹, or both R⁸ and R⁹, may each together with R⁵ or R⁶ form a heterocyclyl or a heteroaryl having 5 to 7 members including a nitrogen atom to which R⁸ or R⁹ is bonded, and the heterocyclyl or the heteroaryl may have a substituent selected from the group consisting of an alkyl having 1 to 6 carbon atoms, an alkenyl having 2 to 6 carbon atoms, an alkynyl having 2 to 6 carbon atoms, an aralkyl group having 6 to 10 carbon atoms, and an alkyl-substituted alkenyl group having 6 to 10 carbon atoms, in which the water-soluble substituent may be bonded directly to the heterocyclyl and the heteroaryl or via the substituent,
wherein R^{a} and R^{b} each independently are a substituted or unsubstituted alkyl group having 1 to 6 carbon atoms, or a substituted or unsubstituted aryl group, in which the alkyl group and the aryl group of R^{a} and R^{b} may be substituted with the water-soluble substituent. In a more preferred embodiment, X may be one selected from the groups represented by the formulas (VIII) and (IX). In an even more preferred embodiment, X may be one selected from the group represented by the formula (VIII). In another even more preferred embodiment, X may be one selected from the group represented by the following formulas (VIII') to (XIII'):. 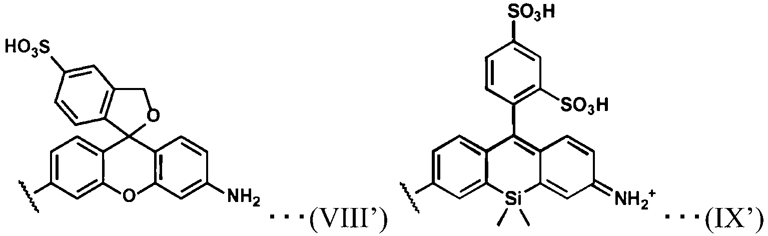 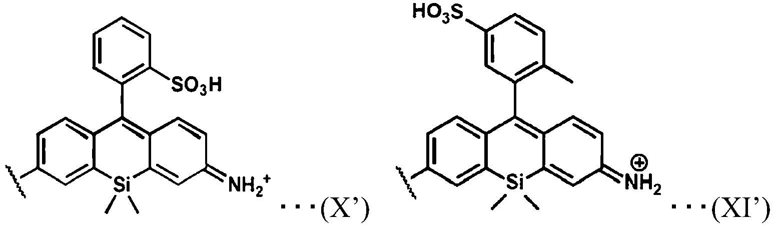 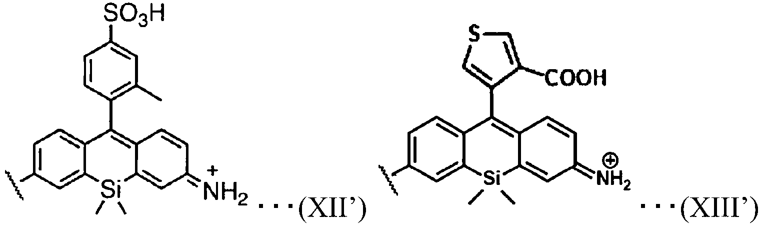
In a yet more preferred embodiment, X may be a group represented by the formula (VIII). In the present disclosure, a compound represented by the following formula (XIV), which is represented by the formula (III) and in which X in the formula is a group represented by the formula (VIII') is also described as Q-sHMRG. 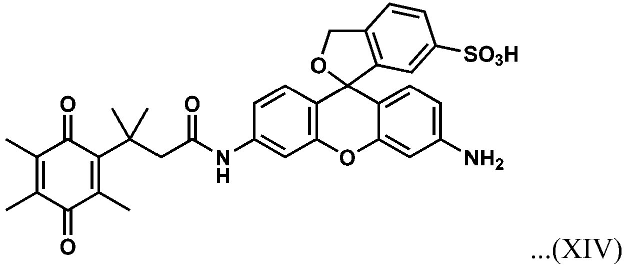 A product of an enzymatic reaction (a reduction reaction) by an NADH:quinone oxidoreductase of Q-sHMRG is the above-described sHMRG.

The fluorescent probes described above can be synthesized by organic synthesis according to methods commonly performed by those skilled in the art.

When Step (B) (a reaction step) of the first aspect of the present disclosure starts, a concentration of a fluorescent probe is not particularly limited as long as it is a concentration at which fluorescence can be detected in a subsequent Step (C) (a fluorescence measurement step), but for example may be 1 µM to 1000 µM, and may also be 3 µM to 700 µM, 10 µM to 500 µM, or 30 µM to 300 µM.

### <Evaluation system>

An evaluation method for an oxidative activity of a dehydrogenase according to the first aspect of the present disclosure comprises the following steps (A) to (C):
Step (A): a preparation step of preparing an aqueous solution containing a single molecule of the dehydrogenase, a substrate of the dehydrogenase, a nucleoside diphosphate derivative, a DT-diaphorase, and a fluorescent probe;
Step (B): a reaction step of causing the dehydrogenase and the DT-diaphorase to catalyze a redox reaction in the aqueous solution prepared in Step (A); and
Step (C): a fluorescence measurement step of measuring fluorescence of the aqueous solution resulting from Step (B).
An evaluation system according to this evaluation method is not particularly limited as long as it can form a closed reaction system by an aqueous solution containing a single molecule of the dehydrogenase, the substrate of the dehydrogenase, a nucleoside diphosphate derivative, a DT-diaphorase, and a fluorescent probe, and is a system in which fluorescence derived from a reduction product of the fluorescent probe generated in the closed reaction system can be quantitatively measured. The closed reaction system may be, for example, spatially closed by a resin or an oil, and a volume of the closed reaction system may be, for example, 5 fL to 100 fL. Also, a plurality of the closed reaction systems may be present, and Step (B) may be performed in each of them. As a more specific evaluation system, for example, an evaluation system using a microdevice provided with wells having a volume of about 5 fL to 100 fL per well may be used, and, at this time, among surfaces of the aqueous solution (the closed reaction system) in Step (B), a portion not covered by a well may be covered with an organic solvent, or may be covered with a gas (for example, air). When a portion not covered by a well among surfaces of the aqueous solution in Step (B) is covered with a gas, the aqueous solution may remain in a well by, for example, gravity and/or surface tension. As another specific evaluation system, an evaluation system using droplets (for example, liposomes) each having a volume of about 5 fL to 100 fL present in an aqueous solution may also be used, and, in this case, by an aqueous solution containing a single molecule of the dehydrogenase, the substrate of the dehydrogenase, a nucleoside diphosphate derivative, a DT-diaphorase, and a fluorescent probe being enclosed in each droplet, an oxidative activity of each dehydrogenase can be evaluated.

In a preferred embodiment of the evaluation method for an oxidative activity of a dehydrogenase according to the first aspect of the present disclosure, an evaluation system is an evaluation system using a microdevice provided with wells having a volume of about 5 fL to 100 fL per well, in which among surfaces of the aqueous solution (the closed reaction system) in Step (B), a portion not covered by a well is covered with an organic solvent, and, hereinafter, the evaluation system is referred to as "an oil-seal chamber system." Such an evaluation system is described, for example, in Patent Literature 1 and Non Patent Literature 1. In an oil-seal chamber system, a closed reaction system can be achieved by using a property that a hydrophobic solvent and an aqueous solution do not mix with each other. As a hydrophobic solvent, it is not particularly limited as long as it does not mix with an aqueous solution, but at least one selected from the group consisting of saturated hydrocarbons, unsaturated hydrocarbons, aromatic hydrocarbons, silicone oils, perfluorocarbons, halogenated solvents, and hydrophobic ionic liquids, or a mixture containing the same, can be suitably used.

### <Step (A): Preparation Step>

In Step (A), an aqueous solution containing a single molecule of the dehydrogenase, the substrate of the dehydrogenase, a nucleoside diphosphate derivative, a DT-diaphorase, and a fluorescent probe is prepared. In Step (A), usually, first a solution (a pre-encapsulation aqueous solution) containing the dehydrogenase, the substrate of the dehydrogenase, a nucleoside diphosphate derivative, a DT-diaphorase, and a fluorescent probe is prepared, and then the obtained pre-encapsulation aqueous solution is enclosed in a microdevice or a droplet or the like so that the dehydrogenase in one closed reaction system becomes a single molecule. That is, in one embodiment, Step (A) may include enclosing, in a space closed to an aqueous solution, a pre-encapsulation aqueous solution containing the dehydrogenase, the substrate of the dehydrogenase, a nucleoside diphosphate derivative, a DT-diaphorase, and a fluorescent probe so that the dehydrogenase in each aqueous solution becomes a single molecule.

When Step (A) comprises enclosing, in a space closed to an aqueous solution, a pre-encapsulation aqueous solution containing the dehydrogenase, the substrate of the dehydrogenase, a nucleoside diphosphate derivative, a DT-diaphorase, and a fluorescent probe so that the dehydrogenase in each aqueous solution becomes a single molecule, and comprises enclosing the aqueous solution in many (for example, 1000 or more) closed spaces, a number-average number of molecules of the dehydrogenase contained per one of the aqueous solutions may be, for example, 0.0002 to 0.5, and may also be 0.0005 to 0.1, 0.001 to 0.07, or 0.003 to 0.05. When the number-average number of molecules of the dehydrogenase contained per one aqueous solution is within the above range, among those aqueous solutions, a possibility that a number of the dehydrogenase in an aqueous solution in which the dehydrogenase is enclosed becomes a single molecule is increased, and therefore it becomes easy to suppress fluorescence derived from an aqueous solution in which two or more molecules of the dehydrogenase are enclosed from being measured in Step (C). In that case, evaluation of an oxidative activity of the dehydrogenase becomes easy. The number-average number of molecules of the dehydrogenase contained per one of the above aqueous solutions may be, for example, a value obtained by multiplying a concentration [molecules/L] of the dehydrogenase in the pre-encapsulation aqueous solution by a volume [L] of a Step (B) aqueous solution (for example, a volume of a well).

Preparation of a pre-encapsulation aqueous solution containing the dehydrogenase, the substrate of the dehydrogenase, a nucleoside diphosphate derivative, a DT-diaphorase, and a fluorescent probe is usually performed by adding the dehydrogenase, the substrate of the dehydrogenase, a nucleoside diphosphate derivative, a DT-diaphorase, and a fluorescent probe to a solvent. At this time, the fluorescent probe and the nucleoside diphosphate derivative may be added in a form of a salt, a solvate, or a solvate of a salt, for example. Also, because when a reduced form of a nucleoside diphosphate derivative is present, a reduction reaction by a DT-diaphorase occurs and a background signal is generated regardless of presence or absence of an oxidation reaction by a dehydrogenase, the nucleoside diphosphate derivative is usually added in an oxidized form. A ratio of a mass of an oxidized form to a sum of masses of the oxidized form and a reduced form in the nucleoside diphosphate derivative to be added is preferably 80% or more, 90% or more, 95% or more, 97% or more, 98% or more, 98.5% or more, 99.0% or more, 99.5% or more, 99.8% or more, or 100%.

A concentration of the dehydrogenase in the pre-encapsulation aqueous solution is not particularly limited as long as it is a concentration at which fluorescence can be detected in a subsequent Step (C) (a fluorescence measurement step), but for example may be 5 fM to 20 pM, and may also be 10 fM to 10 pM, 30 fM to 3 pM, or 100 fM to 1 pM.

Also, for example, in one embodiment of the present disclosure, when the dehydrogenase is a dehydrogenase contained in plasma, as a dilution factor of plasma in the pre-encapsulation aqueous solution, it is not particularly limited as long as it is a dilution factor at which fluorescence can be detected in a subsequent Step (C) (a fluorescence measurement step), but for example may be 10-fold to 1,000,000-fold, and may also be 30-fold to 300,000-fold, 100-fold to 100,000-fold, 300-fold to 30,000-fold, or 500-fold to 10,000-fold.

Also, concentrations of the substrate of the dehydrogenase, the nucleoside diphosphate derivative, the DT-diaphorase, and the fluorescent probe in the pre-encapsulation aqueous solution are usually the same as concentrations of the substrate of the dehydrogenase, the nucleoside diphosphate derivative, the DT-diaphorase, and the fluorescent probe in the aqueous solution prepared in Step (A), and concentrations of the substrate of the dehydrogenase, the nucleoside diphosphate derivative, the DT-diaphorase, and the fluorescent probe in the aqueous solution at a start of Step (B).

A pH of the aqueous solution prepared in Step (A) is not limited as long as it is a pH at which enzymatic reactions by a dehydrogenase and a DT-diaphorase occur, and may be, for example, a pH within a range of ±3.0 of an optimal pH of these dehydrogenase and within a range of ±3.0 of an optimal pH of the DT-diaphorase, a pH within a range of ±2.0 of an optimal pH of these dehydrogenase and within a range of ±2.0 of an optimal pH of the DT-diaphorase, a pH within a range of ±1.5 of an optimal pH of these dehydrogenase and within a range of ±1.5 of an optimal pH of the DT-diaphorase, or a pH within a range of ±1.0 of an optimal pH of these dehydrogenase and within a range of ±1.0 of an optimal pH of the DT-diaphorase. Specifically, a pH of the aqueous solution prepared in Step (A) may be, for example, 3.5 or more, 4.0 or more, 4.5 or more, 5.0 or more, 5.5 or more, 6.0 or more, 6.5 or more, 7.0 or more, 7.5 or more, 8.0 or more, 8.5 or more, or 9.0 or more, and may also be 9.5 or less, 9.0 or less, 8.5 or less, 8.0 or less, 7.5 or less, 7.0 or less, 6.5 or less, 6.0 or less, 5.5 or less, 5.0 or less, 4.5 or less, or 4.0 or less.

Also, the aqueous solution prepared in Step (A) may contain other components in addition to the dehydrogenase, the substrate of the dehydrogenase, the nucleoside diphosphate derivative, the DT-diaphorase, and the fluorescent probe. Examples of other components include a buffer (for example, a phosphate buffer, a HEPES buffer, and a Tris buffer), a pH adjuster (for example, hydrochloric acid, citric acid, sodium hydroxide, sodium carbonate, and sodium hydrogen carbonate), an ion concentration adjuster (for example, calcium chloride and magnesium chloride), an osmotic pressure adjuster (for example, sodium chloride), an antioxidant (for example, dithiothreitol (DTT)), and a surfactant (for example, Triton (registered trademark) X-100, CHAPS, and Tween (registered trademark) 20).

In one embodiment of the first aspect of the present disclosure, when an evaluation system is an oil-seal chamber system, Step (A) may be a step of filling, into a plurality of accommodating portions of a microchamber array whose surface has hydrophobicity, an aqueous solution containing the dehydrogenase, the substrate of the dehydrogenase, the nucleoside diphosphate derivative, the DT-diaphorase, and the fluorescent probe, the step including, in this order, pouring, from openings of the accommodating portions, the aqueous solution containing the dehydrogenase, the substrate of the dehydrogenase, the nucleoside diphosphate derivative, the DT-diaphorase, and the fluorescent probe so that a number-average number of molecules of the dehydrogenase filled in each of the plurality of accommodating portions is 0.0002 to 0.5, and closing the openings with a hydrophobic solvent. As such a microchamber array, for example, those described in Patent Literature 1 and Non Patent Literature 1 can be used, and, more specifically, for example, Simoa (registered trademark) disk (Quanterix) can be used. A volume of each solution in the plurality of accommodating portions may be, for example, 1 fL to 500 fL, 5 fL to 100 fL, 10 fL to 75 fL, or 20 fL to 60 fL. Also, a number-average number of molecules of the dehydrogenase filled in each of the plurality of accommodating portions may be, for example, 0.0002 to 0.5, and may also be 0.0005 to 0.1, 0.001 to 0.07, or 0.003 to 0.05.

In one embodiment of the first aspect of the present disclosure, when an evaluation system is an oil-seal chamber system, from a viewpoint of suppressing leakage into a hydrophobic solvent, a fluorescent probe whose water solubility of the fluorescent probe and its reduction product is high can be suitably used as a fluorescent probe. Examples of such a fluorescent probe include a fluorescent probe having at least one substituent selected from the group consisting of -CO₂H, - PO₃H₂, and -SO₃H, and a fluorescent probe in which, regarding the fluorescent probe and its reduction product, when 1 µM of a compound is added to a two-phase system of octanol and phosphate-buffered saline (PBS) at pH 7.4 at a volume ratio of 1:1, a proportion of the compound distributed in a PBS layer is 80% or more.

In one embodiment of the first aspect of the present disclosure, when an evaluation system is an oil-seal chamber system, from a viewpoint of making fluorescence easier to observe, a fluorescent probe whose product of a molar extinction coefficient [L/mol·cm] at a maximum absorption wavelength and a fluorescence quantum yield of a reduction product is 10000 or more can be suitably used. From the same viewpoint, a fluorescent probe whose maximum fluorescence wavelength of a reduction product is 450 nm or more can be suitably used.

### <Step (B): Reaction Step>

In Step (B), the dehydrogenase and the DT-diaphorase are caused to catalyze a redox reaction in the aqueous solution prepared in Step (A).

A reaction temperature in Step (B) is not particularly limited as long as it is a temperature at which enzymatic reactions by the dehydrogenase and the DT-diaphorase occur, and may be, for example, 20°C or higher and 50°C or lower, and may be 23°C or higher and 40°C or lower. Specific examples of a reaction temperature in Step (B) include, for example, 25°C and 37°C.

A reaction time in Step (B) is not particularly limited as long as it is a time at which fluorescence can be quantitatively measured in a subsequent Step (C) (a fluorescence measurement step), and may be, for example, 1 minute or longer and 72 hours or shorter, and may be 5 minutes or longer and 48 hours or shorter, 10 minutes or longer and 30 hours or shorter, 20 minutes or longer and 24 hours or shorter, or 30 minutes or longer and 8 hours or shorter. Specific examples of a reaction time in Step (B) include, for example, 1 hour, 3 hours, 6 hours, and 12 hours.

### <Step (C): Fluorescence Measurement Step>

In Step (C), fluorescence of the aqueous solution resulting from Step (B) is measured, and fluorescence data are acquired. Step (C) may be performed at the same time as Step (B), and, in this case, Step (C) may be performed multiple times in a course of performing Step (B) (that is, a so-called time-lapse measurement may be performed). Also, a method of measuring fluorescence is not particularly limited as long as it is a method capable of measuring fluorescence of the aqueous solution resulting from Step (B), and may be, for example, a method using a fluorescence microscope, and the fluorescence microscope may be, for example, an epifluorescence microscope, a confocal microscope, or a total internal reflection microscope.

Based on fluorescence data acquired by the method described above, an oxidative activity of a dehydrogenase can be evaluated. An evaluation of an oxidative activity of a dehydrogenase may use, as an index, a number of aqueous solutions, among aqueous solutions subjected to fluorescence measurement, in which a fluorescence intensity equal to or greater than a threshold is observed, may use, as an index, a distribution (a pattern) of fluorescence intensities of aqueous solutions subjected to fluorescence measurement, or may use another index.

Specifically, for example, in a case where, in an aqueous solution containing a dehydrogenase that meets a specific condition (for example, a condition in which a gene mutation has occurred, or a condition in which a post-translational modification is present), a fluorescence intensity becomes equal to or greater than a threshold, while, in an aqueous solution containing a dehydrogenase that does not meet the specific condition (for example, a condition in which a gene mutation has not occurred, or a condition in which a post-translational modification is not present), a fluorescence intensity becomes less than the threshold, the oxidative activity of the dehydrogenase may be evaluated on a basis of whether or not the fluorescence intensity becomes equal to or greater than the threshold, and a dehydrogenase whose fluorescence intensity becomes equal to or greater than the threshold can be evaluated as a dehydrogenase that meets the specific condition.

Also, specifically, for example, in a case where, in an aqueous solution containing a dehydrogenase derived from a sample that meets a specific condition, an average value of fluorescence intensities of aqueous solutions becomes equal to or greater than a threshold, while, in an aqueous solution containing a dehydrogenase derived from a sample that does not meet the specific condition, an average value of fluorescence intensities of aqueous solutions becomes less than the threshold, whether or not an average value of fluorescence intensities in an aqueous solution containing a dehydrogenase derived from a target sample becomes equal to or greater than the threshold may be used as a basis to evaluate the sample itself through evaluation of an oxidative activity of the dehydrogenase, and, for example, a sample in which an average value of fluorescence intensities becomes equal to or greater than the threshold can be evaluated as a sample that meets the specific condition. In this case, for example, when a sample that meets the specific condition is a specimen derived from a cancer patient and a sample that does not meet the specific condition is a specimen derived from a healthy subject, whether or not an average value of fluorescence intensities in an aqueous solution containing a dehydrogenase derived from the sample becomes equal to or greater than the threshold may be used as a basis to assist diagnosis of whether or not the subject suffers from cancer, and data to be used for determining whether or not the subject suffers from cancer can be acquired.

Also, for example, in a case where, in a sample that meets a specific condition, a proportion of aqueous solutions in which a fluorescence intensity becomes equal to or greater than a threshold becomes equal to or greater than a predetermined value, while, in a sample that does not meet the specific condition, a proportion of aqueous solutions in which a fluorescence intensity becomes equal to or greater than the threshold becomes less than the predetermined value, whether or not a proportion of aqueous solutions in which a fluorescence intensity becomes equal to or greater than the threshold in a target sample becomes equal to or greater than the predetermined value may be used as a basis to evaluate the sample itself through evaluation of an oxidative activity of the dehydrogenase, and, for example, a sample in which a proportion of aqueous solutions in which a fluorescence intensity becomes equal to or greater than the threshold becomes equal to or greater than the predetermined value can be evaluated as a sample that meets the specific condition. In this case, for example, when a sample that meets the specific condition is a specimen derived from a cancer patient and a sample that does not meet the specific condition is a specimen derived from a healthy subject, whether or not a proportion of aqueous solutions in which a fluorescence intensity becomes equal to or greater than the threshold in the sample becomes equal to or greater than the predetermined value may be used as a basis to assist diagnosis of whether or not the subject suffers from cancer, and data to be used for determining whether or not the subject suffers from cancer can be acquired.

Also, specifically, for example, in a case where a distribution (a pattern) of fluorescence intensities in an aqueous solution containing a dehydrogenase derived from a sample that meets a specific condition and a distribution (a pattern) of fluorescence intensities in an aqueous solution containing a dehydrogenase derived from a sample that does not meet the specific condition are different, whether or not a distribution (a pattern) of fluorescence intensities in an aqueous solution containing a dehydrogenase derived from a target sample is more similar to either distribution may be used as a basis to evaluate the sample itself through evaluation of an oxidative activity of the dehydrogenase, and, for example, when a p-value obtained when a Student's t-test is performed between a sample that meets the specific condition and a target sample is compared with a p-value obtained when a Student's t-test is performed between a sample that does not meet the specific condition and the target sample, when the p-value obtained when a Student's t-test is performed between the sample that meets the specific condition and the target sample is smaller, the sample can be evaluated as a sample that meets the specific condition. In this case, for example, when a sample that meets the specific condition is a specimen derived from a cancer patient and a sample that does not meet the specific condition is a specimen derived from a healthy subject, whether or not the p-value obtained when a Student's t-test is performed between the sample that meets the specific condition and the target sample is larger may be used as a basis to assist diagnosis of whether or not the subject suffers from cancer, and data to be used for determining whether or not the subject suffers from cancer can be acquired.

Also, specifically, for example, when fluorescence data are fluorescence images (for example, fluorescence images acquired by an evaluation in an oil-seal chamber system), by using a machine learning model trained such that a fluorescence image obtained when a sample that meets a specific condition is used is treated as a sample that meets the specific condition and a fluorescence image obtained when a sample that does not meet the specific condition is used is treated as a sample that does not meet the specific condition, a fluorescence image in a target sample may be input to have the machine learning model determine whether or not the target sample meets the specific condition, whereby the sample itself can be evaluated through evaluation of an oxidative activity of the dehydrogenase.

### <Effect and other embodiments>

According to one embodiment of the present disclosure, an oxidative activity of a dehydrogenase can be evaluated (detected) at the single-molecule level of a dehydrogenase.

According to one embodiment of the present disclosure, even when one kind of fluorescent probe is used, it is possible to detect (evaluate) oxidative activities of multiple types of dehydrogenases by using various nucleoside diphosphate derivatives. According to one embodiment of the present disclosure, even when one kind of fluorescent probe is used, it is possible to detect (evaluate) oxidative activities of multiple types of dehydrogenases by using various nucleoside diphosphate derivatives. Another embodiment of the first aspect of the present disclosure is a method for evaluating an oxidative activity of a dehydrogenase, comprising the following steps (A-1) to (C-1) and steps (A-2) to (C-2):
Step (A-1): a step of preparing an aqueous solution containing a single molecule of the dehydrogenase, a substrate of the dehydrogenase, a first nucleoside diphosphate derivative, a DT-diaphorase, and a fluorescent probe;
Step (B-1): a step of causing the dehydrogenase and the DT-diaphorase to catalyze a redox reaction in the aqueous solution prepared in Step (A-1);
Step (C-1): a step of measuring fluorescence of the aqueous solution resulting from Step (B-1);
Step (A-2): a step of preparing an aqueous solution containing a single molecule of the dehydrogenase, the substrate of the dehydrogenase, a second nucleoside diphosphate derivative, the DT-diaphorase, and the fluorescent probe;
Step (B-2): a step of causing the dehydrogenase and the DT-diaphorase to catalyze a redox reaction in the aqueous solution prepared in Step (A-2); and
Step (C-2): a step of measuring fluorescence of the aqueous solution resulting from Step (B-2).
In the embodiment, the first nucleoside diphosphate derivative is different from the second nucleoside diphosphate derivative (i.e., both have different chemical structures). In the embodiment, the dehydrogenase is capable of using an oxidized form of the first nucleoside diphosphate derivative and an oxidized form of the second nucleoside diphosphate derivative as a coenzyme. In the embodiment, the DT-diaphorase is capable of using a reduced form of the first nucleoside diphosphate derivative and a reduced form of the second nucleoside diphosphate derivative as a coenzyme. In the embodiment, the fluorescent probe is a compound whose fluorescence intensity increases upon reduction by the DT-diaphorase. According to the embodiment described above, by using one kind of fluorescent probe and two kinds of nucleoside diphosphate derivatives (a first nucleoside diphosphate derivative and a second nucleoside diphosphate derivative), it is possible to detect and distinguish oxidative activities of multiple types of dehydrogenases that metabolize the same substrate by utilizing a difference in utilization efficiency by the dehydrogenase according to a type of the nucleoside diphosphate derivative. For example, in a case where a dehydrogenase A is capable of using the first nucleoside diphosphate derivative as coenzymes and a dehydrogenase B is capable of using the second nucleoside diphosphate derivative as coenzymes, respectively, it is possible to interpret a result measured under a condition in which the first nucleoside diphosphate derivative is added for a certain sample as an oxidative activity of the dehydrogenase A, and it is possible to interpret a result measured under a condition in which the second nucleoside diphosphate derivative is added for the same sample as an oxidative activity of the dehydrogenase B.

According to one embodiment of the present disclosure, even when one kind of fluorescent probe is used, it is possible to detect (evaluate) oxidative activities of multiple types of dehydrogenases by using substrates of various dehydrogenases. According to one embodiment of the present disclosure, even when one kind of fluorescent probe is used, it is possible to detect (evaluate) oxidative activities of multiple types of dehydrogenases by using substrates of various dehydrogenases. Another embodiment of the first aspect of the present disclosure is a method for evaluating an oxidative activity of a dehydrogenase, comprising the following steps (A-3) to (C-3) and steps (A-4) to (C-4):
Step (A-3): a step of preparing an aqueous solution containing a single molecule of a first dehydrogenase, a substrate of the first dehydrogenase, a nucleoside diphosphate derivative, a DT-diaphorase, and a fluorescent probe;
Step (B-3): a step of causing the first dehydrogenase and the DT-diaphorase to catalyze a redox reaction in the aqueous solution prepared in Step (A-3);
Step (C-3): a step of measuring fluorescence of the aqueous solution resulting from Step (B-3);
Step (A-4): a step of preparing an aqueous solution containing a single molecule of a second dehydrogenase, a substrate of the second dehydrogenase, the nucleoside diphosphate derivative, the DT-diaphorase, and the fluorescent probe;
Step (B-4): a step of causing the second dehydrogenase and the DT-diaphorase to catalyze a redox reaction in the aqueous solution prepared in Step (A-4); and
Step (C-4): a step of measuring fluorescence of the aqueous solution resulting from Step (B-4).
In the embodiment, the substrate of the first dehydrogenase is different from the substrate of the second dehydrogenase (i.e., both have different chemical structures), the first dehydrogenase and the second dehydrogenase are capable of using an oxidized form of the nucleoside diphosphate derivative as a coenzyme. In the embodiment, the DT-diaphorase is capable of using a reduced form of the nucleoside diphosphate derivative as a coenzyme. In the embodiment, the fluorescent probe is a compound whose fluorescence intensity increases upon reduction by the DT-diaphorase. According to the embodiment described above, by using one kind of fluorescent probe and two kinds of substrates of dehydrogenases (a substrate of the first dehydrogenase and a substrate of the second dehydrogenase), it is possible to detect and distinguish oxidative activities of multiple types of dehydrogenases by utilizing a difference in substrate specificity of dehydrogenases. For example, in a case where a dehydrogenase A can metabolize the substrate of the first dehydrogenase as a substrate and a dehydrogenase B can metabolize the substrate of the second dehydrogenase as a substrate, respectively, it is possible to interpret a result measured under a condition in which the substrate of the first dehydrogenase is added for a certain sample as an oxidative activity of the dehydrogenase A, and it is possible to interpret a result measured under a condition in which the substrate of the second dehydrogenase is added for the same sample as an oxidative activity of the dehydrogenase B.

According to one embodiment of the present disclosure, by utilizing a fact that an oxidative activity changes in a dehydrogenase derived from a sample that meets specific conditions, it is possible to evaluate whether or not the sample meets specific conditions through detecting an enzymatic activity of a dehydrogenase in the sample. In this case, according to one embodiment of the present disclosure, even when one kind of fluorescent probe is used, it is possible to discover a coupled assay system suitable for evaluating such a sample that meets such specific conditions by using substrates of various dehydrogenases and various nucleoside diphosphate derivatives.

### <Kit>

A second aspect of the present disclosure is a kit for evaluating an oxidative activity of a dehydrogenase, comprising a substrate of the dehydrogenase, a nucleoside diphosphate derivative, a DT-diaphorase, and a fluorescent probe, where the dehydrogenase is capable of using an oxidized form of the nucleoside diphosphate derivative as a coenzyme, the DT-diaphorase is capable of using a reduced form of the nucleoside diphosphate derivative as a coenzyme, and the fluorescent probe is a compound whose fluorescence intensity increases upon reduction by the DT-diaphorase, the kit. As the substrate of the dehydrogenase, the nucleoside diphosphate derivative, the DT-diaphorase, and the fluorescent probe according to the second aspect of the present disclosure, those described in the first aspect of the present disclosure can be used, and a dehydrogenase to be evaluated can target the same dehydrogenase as described in the first aspect of the present disclosure. According to the kit according to the second aspect of the present disclosure, an oxidative activity of a dehydrogenase can be evaluated in accordance with the evaluation method according to the first aspect of the present disclosure. The kit according to the second aspect of the present disclosure may further include an attached document describing that an evaluation of an oxidative activity of a dehydrogenase is to be performed, may further include an attached document describing that an evaluation of an oxidative activity of a single molecule of a dehydrogenase is to be performed, and the attached document may describe that steps corresponding to Steps (A) to (C) in the evaluation method according to one embodiment of the first aspect of the present disclosure are to be performed. The kit according to the second aspect of the present disclosure may further include a microchamber array in one embodiment, and the microchamber array may be one that can be used in the evaluation method according to the first aspect of the present disclosure.

### <Modified Embodiment: Method for Evaluating Metabolic Activity of Metabolic Enzyme>

A third aspect of the present disclosure is a method for evaluating a metabolic activity of a metabolic enzyme, comprising the following steps (A) to (C):
Step (A): a step of preparing an aqueous solution containing a single molecule of the metabolic enzyme, a substrate of the metabolic enzyme, a dehydrogenase, a nucleoside diphosphate derivative, a DT-diaphorase, and a fluorescent probe;
Step (B): a step of causing the metabolic enzyme, the dehydrogenase, and the DT-diaphorase to catalyze an enzymatic reaction in the aqueous solution prepared in Step (A); and
Step (C): a step of measuring fluorescence of the aqueous solution resulting from Step (B).
In this aspect, the dehydrogenase is a dehydrogenase that uses, as a substrate, a metabolite resulting from the metabolic enzyme metabolizing the substrate of the metabolic enzyme. In this aspect, the dehydrogenase is capable of using an oxidized form of the nucleoside diphosphate derivative as a coenzyme. In this aspect, the DT-diaphorase is capable of using a reduced form of the nucleoside diphosphate derivative as a coenzyme. In this aspect, and the fluorescent probe is a compound whose fluorescence intensity increases upon reduction by the DT-diaphorase, the evaluation method. That is, the third aspect of the present disclosure is a method of performing a coupled assay shown in the following reaction scheme at the single-molecule level of the metabolic enzyme. In the coupled assay shown in the following reaction scheme, by making an enzymatic reaction by the metabolic enzyme the rate-limiting step of an entire coupled assay system, that is, by making a metabolite generated by the enzymatic reaction of the metabolic enzyme promptly consumed for enzymatic reactions by the dehydrogenase and the DT-diaphorase, it becomes possible to evaluate a metabolic activity of the metabolic enzyme by using fluorescence of a reduction product of the enzymatic reaction by the DT-diaphorase as an index.

Substrate of
Metabolic enzyme
Metabolic enzyme
Metabolite of
Metabolic enzyme
||
Substrate of
Dehydrogenase
Oxidized form of
Nucleoside diphosphate
derivative
Reduced product
(Strongly fluorescent)
Dehydrogenase
DT-diaphorase
Oxidized product
Reduced form of
Nucleoside diphosphate
derivative
Fluorescent probe
(Weakly fluorescent)

As shown in the reaction scheme above as "Metabolite of Metabolic enzyme = Substrate of the Dehydrogenase," the evaluation method according to the third aspect of the present disclosure can also be described as the evaluation method according to the first aspect of the present disclosure in which, in Step (A), an aqueous solution containing a single molecule of a metabolic enzyme and a substrate of the metabolic enzyme is prepared instead of a substrate of a dehydrogenase, and, in Step (B), the metabolic enzyme is also caused to catalyze an enzymatic reaction, where the dehydrogenase is a dehydrogenase that uses, as a substrate, a metabolite resulting from the metabolic enzyme metabolizing the substrate of the metabolic enzyme. Thereby, a metabolite resulting from the metabolic enzyme metabolizing the substrate of the metabolic enzyme functions as "a substrate of the dehydrogenase" in the evaluation method according to the first aspect of the present disclosure.

As the nucleoside diphosphate derivative, the DT-diaphorase, and the fluorescent probe according to the third aspect of the present disclosure, those that can be used in the first aspect of the present disclosure can be used. Also, regarding an evaluation system and conditions of Steps (A) to (C) according to the third aspect of the present disclosure, except for matters specially described below, those that can be used in an evaluation system and conditions of Steps (A) to (C) according to the first aspect of the present disclosure can be used. Also, an evaluation of a metabolic enzyme metabolic activity according to the third aspect of the present disclosure can be performed by the same evaluation method as the evaluation of an oxidative activity of a dehydrogenase described in the first aspect of the present disclosure.

### <Metabolic Enzyme>

In the present disclosure, a metabolic enzyme means an enzyme that is present in a cell or on a cell membrane or is secreted from a cell and has a function of converting a substance into another substance. Typical examples of a metabolic enzyme include enzymes constituting energy-producing pathways represented by a glycolytic pathway, a citric acid cycle, and an electron transport chain, and enzymes involved in amino acid metabolism or lipid metabolism. Examples of a metabolic enzyme according to one embodiment of the third aspect of the present disclosure include enzymes constituting a glycolytic pathway, enzymes involved in glycogen metabolism, enzymes involved in gluconeogenesis, enzymes constituting a citric acid cycle, enzymes constituting a pentose phosphate pathway, enzymes involved in drug metabolism and the like, enzymes involved in metabolism of amino acids and analogs thereof, and enzymes involved in lipid metabolism, and, more specifically, include hexokinase, phosphofructokinase, aldolase, enolase, pyruvate decarboxylase, phosphoglucomutase, fructose bisphosphatase, aconitase, fumarate hydratase, 6-phosphogluconolactonase, glycine decarboxylase, lipid desaturase, and phospholipase. Also, examples of a metabolic enzymes according to one embodiment of the third aspect of the present disclosure include metabolic enzymes that produce, as metabolites by a metabolic reaction, a substrate of dehydrogenases according to the first aspect of the present disclosure (for example, glycine, serine, D-serine, glutamic acid, lysine, leucine, glutathione, glucose-6-phosphate, lactate, D-lactate, isocitrate, malate, inosinic acid, 6-phosphogluconate, succinic semialdehyde, terephthalaldehydic acid, or HMG-CoA).

In one embodiment of the third aspect of the present disclosure, a metabolic enzyme may be a metabolic enzyme contained in at least one selected from the group consisting of plasma, serum, urine, saliva, tears, cerebrospinal fluid, tissue homogenate, cell homogenate, and extracts thereof. In a preferred embodiment, a metabolic enzyme may be a metabolic enzyme contained in at least one selected from the group consisting of plasma, serum, urine, saliva, tears, and cerebrospinal fluid, and extracts thereof, and in an even more preferred embodiment, a metabolic enzyme may be a metabolic enzyme contained in at least one selected from the group consisting of plasma, serum, tears, and cerebrospinal fluid, and extracts thereof. When a sample from which the metabolic enzyme is derived is evaluated through evaluation of a metabolic activity of the metabolic enzyme by the evaluation method according to one embodiment of the third aspect of the present disclosure, the sample may be at least one selected from the group consisting of plasma, serum, urine, saliva, tears, cerebrospinal fluid, tissue homogenate, cell homogenate, and extracts thereof.

In the present disclosure, a substrate of a metabolic enzyme is a molecule that can be a substrate of a metabolic enzyme according to one embodiment of the third aspect of the present disclosure. As a substrate of a metabolic enzyme according to the present disclosure, it may be a natural substrate of a metabolic enzyme, and may also be an artificial substrate such as a derivative of such a natural substrate. A substrate of a metabolic enzyme can be selected in accordance with a type of a metabolic enzyme to be evaluated, and, for example, when a metabolic enzyme is hexokinase, a substrate of the metabolic enzyme may be at least one selected from the group consisting of hexoses such as D-glucose, D-mannose, and D-fructose. Also, for example, when a metabolic enzyme is phosphoglucomutase, a substrate of the metabolic enzyme may be glucose-1-phosphate. Also, for example, when a metabolic enzyme is aconitate hydratase, a substrate of the metabolic enzyme may be citric acid and/or cis-aconitate. Also, for example, when a metabolic enzyme is 6-phosphogluconolactonase, a substrate of the metabolic enzyme may be 6-phosphoglucono-1,5-lactone.

When Step (B) (a reaction step) of the first aspect of the present disclosure starts, a concentration of a substrate of the metabolic enzyme is not particularly limited as long as it is a concentration at which fluorescence can be detected in a subsequent Step (C) (a fluorescence measurement step), and may be, for example, 10 µM to 1 M, and may also be 30 µM to 100 mM, 100 µM to 30 mM, or 300 µM to 10 mM.

### <Dehydrogenase>

As types of dehydrogenases according to the third aspect of the present disclosure, those described in the first aspect of the present disclosure can be used, and, in accordance with the metabolic enzyme and a substrate of the metabolic enzyme, those that can use a metabolite resulting from the substrate of the metabolic enzyme being metabolized by the metabolic enzyme are selected as a substrate. For example, when the metabolic enzyme is hexokinase and a substrate of the metabolic enzyme is D-glucose, as the dehydrogenase, glucose-6-phosphate dehydrogenase (G6PDH), which is a dehydrogenase that uses as a substrate glucose-6-phosphate, which is a metabolite of D-glucose by hexokinase, can be used. Also, for example, when the metabolic enzyme is phosphoglucomutase and a substrate of the metabolic enzyme is glucose-1-phosphate, as the dehydrogenase, glucose-6-phosphate dehydrogenase (G6PDH), which is a dehydrogenase that uses as a substrate glucose-6-phosphate, which is a metabolite of glucose-1-phosphate by phosphoglucomutase, can be used. Also, for example, when the metabolic enzyme is aconitate hydratase and a substrate of the metabolic enzyme is citric acid, as the dehydrogenase, isocitrate dehydrogenase, which is a dehydrogenase that uses as a substrate isocitrate, which is a metabolite of citric acid by aconitate hydratase, can be used. Also, for example, when the metabolic enzyme is 6-phosphogluconolactonase and a substrate of the metabolic enzyme is 6-phosphoglucono-1,5-lactone, as the dehydrogenase, 6-phosphogluconate dehydrogenase, which is a dehydrogenase that uses as a substrate 6-phosphogluconate, which is a metabolite of 6-phosphoglucono-1,5-lactone by 6-phosphogluconolactonase, can be used.

When Step (B) (a reaction step) of the first aspect of the present disclosure starts, a concentration of a dehydrogenase is not particularly limited as long as it is a concentration at which fluorescence can be detected in a subsequent Step (C) (a fluorescence measurement step), and may be, for example, 0.1 µg/mL to 100 µg/mL, and may also be 0.5 µg/mL to 60 µg/mL or 0.8 µg/mL to 30 µg/mL.

When Step (B) (a reaction step) of the first aspect of the present disclosure starts, a reaction rate constant k [/s] of an enzymatic reaction by the dehydrogenase in an aqueous solution, which is defined as a mathematical product of a number of molecules of the dehydrogenase present in the aqueous solution and a turnover number kcat (/s) of the dehydrogenase, is not particularly limited as long as it is a value at which fluorescence can be detected in a subsequent Step (C) (a fluorescence measurement step), and may be, for example, 0.001 or more, may be 0.003 or more, and is preferably 0.01 or more, 0.03 or more, or 0.1 or more. When the reaction rate constant k of the enzymatic reaction by the dehydrogenase in the aqueous solution in Step (B) (the reaction step) is equal to or greater than the lower limit described above, a rate-limiting step in an entire coupled assay tends to be an enzymatic reaction by the metabolic enzyme, and therefore it becomes easier to evaluate an oxidative activity of the metabolic enzyme.

### <Step (A) of the Third Aspect: Preparation Step>

In Step (A) according to the third aspect of the present disclosure, an aqueous solution containing a single molecule of a metabolic enzyme, a substrate of the metabolic enzyme, a dehydrogenase, a nucleoside diphosphate derivative, a DT-diaphorase, and a fluorescent probe is prepared. In Step (A), usually, the metabolic enzyme, the substrate of the metabolic enzyme, the dehydrogenase, the nucleoside diphosphate derivative, the DT-diaphorase, and the fluorescent probe are prepared, and then the obtained pre-encapsulation aqueous solution is enclosed in a microdevice or a droplet or the like so that the metabolic enzyme in one closed reaction system becomes a single molecule. That is, in one embodiment, Step (A) may include enclosing a pre-encapsulation aqueous solution containing the metabolic enzyme, the substrate of the metabolic enzyme, the dehydrogenase, the nucleoside diphosphate derivative, the DT-diaphorase, and the fluorescent probe in a space closed to an aqueous solution so that the metabolic enzyme in each aqueous solution becomes a single molecule.

When Step (A) includes enclosing a pre-encapsulation aqueous solution containing the metabolic enzyme, the substrate of the metabolic enzyme, the dehydrogenase, the nucleoside diphosphate derivative, the DT-diaphorase, and the fluorescent probe in a space closed to an aqueous solution so that the metabolic enzyme in each aqueous solution becomes a single molecule, and includes enclosing the aqueous solution in many (for example, 1000 or more) closed spaces, a number-average number of molecules of the metabolic enzyme contained per one of the aqueous solutions may be, for example, 0.0002 to 0.5, and may also be 0.0005 to 0.1, 0.001 to 0.07, or 0.003 to 0.05. When the number-average number of molecules of the metabolic enzyme contained per one aqueous solution is within the above range, a possibility that a number of the metabolic enzyme in an aqueous solution in which the metabolic enzyme is among those aqueous solutions enclosed becomes a single molecule is increased, and therefore it becomes easy to suppress fluorescence derived from an aqueous solution in which two or more molecules of the metabolic enzyme are enclosed from being measured in Step (C). In that case, evaluation of an oxidative activity of the metabolic enzyme becomes easy. The number-average number of molecules of the metabolic enzyme contained per one of the above aqueous solutions may be, for example, a value obtained by multiplying a concentration [molecules/L] of the metabolic enzyme in the pre-encapsulation aqueous solution by a volume [L] of a Step (B) aqueous solution (for example, a volume of a well).

A concentration of the metabolic enzyme in the pre-encapsulation aqueous solution is not particularly limited as long as it is a concentration at which fluorescence can be detected in a subsequent Step (C) (a fluorescence measurement step), and may be, for example, 5 fM to 20 pM, and may also be 10 fM to 10 pM, 30 fM to 3 pM, or 100 fM to 1 pM.

Also, for example, in one embodiment of the present disclosure, when the metabolic enzyme is a metabolic enzyme contained in plasma, a dilution factor of plasma in the pre-encapsulation aqueous solution is not particularly limited as long as it is a dilution factor at which fluorescence can be detected in a subsequent Step (C) (a fluorescence measurement step), and may be, for example, 10-fold to 1,000,000-fold, and may also be 30-fold to 300,000-fold, 100-fold to 100,000-fold, 300-fold to 30,000-fold, or 500-fold to 10,000-fold.

Also, concentrations of the substrate of the metabolic enzyme, the dehydrogenase, the nucleoside diphosphate derivative, the DT-diaphorase, and the fluorescent probe in the pre-encapsulation aqueous solution are usually the same as concentrations of the substrate of the metabolic enzyme, the dehydrogenase, the nucleoside diphosphate derivative, the DT-diaphorase, and the fluorescent probe in the aqueous solution prepared in Step (A), and concentrations of the substrate of the metabolic enzyme, the dehydrogenase, the nucleoside diphosphate derivative, the DT-diaphorase, and the fluorescent probe in the aqueous solution at a start of Step (B).

Also, the aqueous solution prepared in Step (A) may contain other components in addition to the metabolic enzyme, the substrate of the metabolic enzyme, the dehydrogenase, the nucleoside diphosphate derivative, the DT-diaphorase, and the fluorescent probe. Examples of other components include the other components explained in the first aspect of the present disclosure, and components consumed by a metabolic reaction by the metabolic enzyme. Examples of components consumed by a metabolic reaction by the metabolic enzyme include adenosine triphosphate (ATP) and adenosine diphosphate (ADP).

In one embodiment of the third aspect of the present disclosure, when an evaluation system is an oil-seal chamber system, Step (A) may be a step of filling, into a plurality of accommodating portions of a microchamber array whose surface has hydrophobicity, an aqueous solution containing the metabolic enzyme, the substrate of the metabolic enzyme, the dehydrogenase, the nucleoside diphosphate derivative, the DT-diaphorase, and the fluorescent probe, the step including, in this order, pouring, from openings of the accommodating portions, the aqueous solution containing the metabolic enzyme, the substrate of the metabolic enzyme, the dehydrogenase, the nucleoside diphosphate derivative, the DT-diaphorase, and the fluorescent probe so that a number-average number of molecules of the metabolic enzyme filled in each of the plurality of accommodating portions is 0.0002 to 0.5, and closing the openings with a hydrophobic solvent. As such a microchamber array, for example, those described in Patent Literature 1 and Non Patent Literature 1 can be used, and, more specifically, for example, Simoa (registered trademark) disk (Quanterix) can be used. A volume of each solution in the plurality of accommodating portions may be, for example, 1 fL to 500 fL, 5 fL to 100 fL, 10 fL to 75 fL, or 20 fL to 60 fL. Also, a number-average number of molecules of the metabolic enzyme filled in each of the plurality of accommodating portions may be, for example, 0.0002 to 0.5, and may also be 0.0005 to 0.1, 0.001 to 0.07, or 0.003 to 0.05.

### <Step (B) of the Third Aspect: Reaction Step>

In Step (B), in the aqueous solution prepared in Step (A), an enzymatic reaction is caused to occur by the metabolic enzyme, the dehydrogenase, and the DT-diaphorase. As conditions in Step (B) of the third aspect of the present disclosure, those in the first aspect of the present disclosure can be used.

<Step (C) of the Third Aspect: Fluorescence Measurement Step> In Step (C), fluorescence of the aqueous solution resulting from Step (B) is measured, and fluorescence data are acquired. As conditions in Step (C) of the third aspect of the present disclosure, those in the first aspect of the present disclosure can be used.

### <Kit for Evaluating Activity of Metabolic Enzyme>

A fourth aspect of the present disclosure is a kit for evaluating a metabolic activity of a metabolic enzyme, comprising a substrate of the metabolic enzyme, a dehydrogenase, a nucleoside diphosphate derivative, a DT-diaphorase, and a fluorescent probe. In this aspect, the dehydrogenase is a dehydrogenase that uses, as a substrate, a metabolite resulting from the substrate of the metabolic enzyme being metabolized by the metabolic enzyme. In this aspect, the dehydrogenase is capable of using an oxidized form of the nucleoside diphosphate derivative as a coenzyme. In this aspect, the DT-diaphorase is capable of using a reduced form of the nucleoside diphosphate derivative as a coenzyme. In this aspect, the fluorescent probe is a compound whose fluorescence intensity increases upon reduction by the DT-diaphorase, the kit. As the substrate of the metabolic enzyme, the dehydrogenase, the nucleoside diphosphate derivative, the DT-diaphorase, and the fluorescent probe according to the fourth aspect of the present disclosure, those described in the third aspect of the present disclosure can be used, and a metabolic enzyme to be evaluated can be the same metabolic enzyme as described in the third aspect of the present disclosure. According to the kit according to the fourth aspect of the present disclosure, a metabolic activity of a metabolic enzyme can be evaluated in accordance with the evaluation method according to the third aspect of the present disclosure. The kit according to the fourth aspect of the present disclosure may further include an attached document describing that an evaluation of an oxidative activity of a metabolic enzyme is to be performed, may further include an attached document describing that an evaluation of an oxidative activity of a single molecule of a metabolic enzyme is to be performed, and the attached document may describe that steps corresponding to Steps (A) to (C) in the evaluation method according to one embodiment of the third aspect of the present disclosure are to be performed. The kit according to the fourth aspect of the present disclosure may further include a microchamber array in one embodiment, and the microchamber array may be one that can be used in the evaluation method according to the third aspect of the present disclosure.

### Examples

Hereinafter, the present disclosure will be described more specifically with reference to examples; however, the present disclosure is not limited to the examples.

### <Synthesis Example 1: Q-sHMRG>

A fluorescent probe Q-sHMRG used in the examples was synthesized as follows.

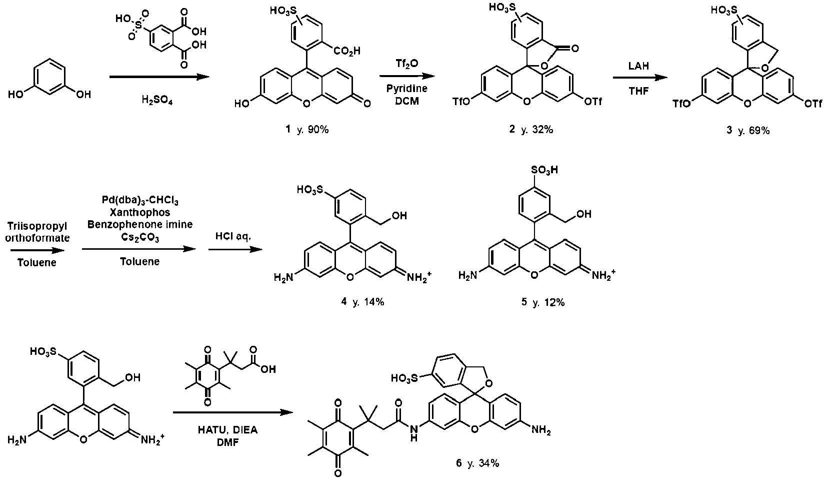

### Synthesis Example 1-1: Preparation of fluorescein 5/6-sulfonic acid (Compound 1)

An aqueous solution of 4-sulfophthalic acid (50%, 20 mL, 40.7 mmol) was mixed with resorcinol (8.95 g, 81.3 mmol), and methanesulfonic acid (2.5 mL) was added. The resulting reaction solution was stirred at 130°C for 18 hours. H₂O (50 mL) was added to the reaction solution, the yellow precipitate was filtered, and washed several times with H₂O. By drying the precipitate, a crude product of fluorescein sulfonic acid 5/6-isomer (15.2 g, 91%, Compound 1) was obtained.

### Synthesis Example 1-2: Preparation of 3-oxo-3',6'-bis(((trifluoromethyl)sulfonyl)oxy)-3H-spiro[isobenzofuran-1,9'-xanthene]-6-sulfonic acid (Compound 2)

The 5/6-isomer of fluorescein sulfonic acid (1.7 g, 4.1 mmol) obtained in Synthesis Example 1-1 was dissolved in DMF (10 mL) and N,N-diisopropylethylamine (DIEA, 10.7 mL, 61.8 mmol). To the resulting mixture, on ice, N-phenylbis(trifluoromethanesulfonimide) (2.9 g, 8.3 mmol) was added as a solid in portions, and the reaction mixture was stirred at 25°C for 2 hours. After confirming complete consumption of the starting material, DIEA was removed under reduced pressure, and dichloromethane (DCM) was added to the remaining solution. The organic layer was washed twice with 2 N HCl aqueous solution and once with saturated brine, dried over Na₂SO₄, filtered, and the solvent was removed under reduced pressure. The residue was purified by MPLC (a gradient of A/B = 90/10 to 0/100 over 15 minutes; where A is 0.1% trifluoroacetic acid (TFA) and B is 80% acetonitrile (ACN)-20% H₂O containing 0.1% TFA) to afford Compound 2 (2.4 g, 86%).

### Synthesis Example 1-3: Preparation of 3',6'-bis(((trifluoromethyl)sulfonyl)oxy)-3H-spiro[isobenzofuran-1,9'-xanthene]-5/6-sulfonic acid (Compound 3)

Compound 2 (2.0 g, 3.0 mmol) obtained in Synthesis Example 1-2 was dissolved in dry THF (30 mL). The resulting solution was placed under an argon atmosphere and cooled to -20°C. A THF solution of lithium aluminum hydride (LAH) (2 M, 4.4 mL, 8.8 mmol) was then added dropwise, and the mixture was warmed to 25°C and stirred for 2 hours. The reaction solution was slowly poured into ice-cooled 2 N HCl aqueous solution (50 mL), and dichloromethane (100 mL) was added. The organic layer was collected, washed with saturated brine, dried over Na₂SO₄, filtered, and the solvent was removed under reduced pressure. The residue was purified by MPLC (a gradient of A/B = 90/10 to 0/100 over 15 minutes; where A is 0.1% trifluoroacetic acid (TFA) and B is 80% acetonitrile (ACN)-20% H₂O containing 0.1% TFA) to afford Compound 3 (1.35 g, 69%).

### Synthesis Example 1-4: Preparation of 3',6'-diamino-3H-spiro[isobenzofuran-1,9'-xanthene]-5/6-sulfonic acid (sHMRG) 6-isomer (Compound 4)

Compound 3 (500 mg, 0.74 mmol) obtained in Synthesis Example 1-3 was dissolved in toluene (30 mL) and triisopropyl orthoformate (1.6 mL, 7.4 mmol), and the reaction mixture was stirred at 25°C for 30 minutes. The solvent was removed under reduced pressure, and the residue was re-dissolved in 10 mL of toluene. To this were added benzophenone imine (633 mg, 3.8 mmol), Pd₂(dba)₃-CHCl₃ (117 mg, 0.11 mmol), xantphos (87 mg, 0.20 mmol), and Cs₂CO₃ (2.46 g, 7.6 mmol), and the reaction mixture was refluxed for 18 hours (105°C) under an argon atmosphere. The solvent of the reaction solution was removed under reduced pressure, and saturated hydrochloric acid aqueous solution (10 mL) was added to the residue. After stirring for 30 minutes, the reaction mixture was diluted in DMSO-H₂O, and directly subjected to preparative purification. Preparative purification was performed by collecting a fraction having an absorption band with an absorption peak around 500 nm in MPLC (a gradient of A/B = 99/1 to 0/100 over 15 minutes; where A is 0.1 M triethylamine acetate buffer and B is methanol). The earlier-eluting isomer was designated as the 6-sulfonic acid isomer (Compound 4, 41 mg, 14%), and the later-eluting isomer was designated as the 5-sulfonic acid isomer (Compound 5, 36 mg, 12%).

### Compound 4

¹H-NMR (DMSO-d6): δ 4.13 (s, 2H), 6.73 (d, 2H, J = 2.0 Hz), 6.83 (dd, 2H, J = 2.0, 9.2 Hz), 6.95 (d, 2H, J = 9.2 Hz), 7.38 (d, 1H, J = 1.6 Hz), 7.64 (d, 1H, J = 8.8 Hz), 7.78 (dd, 1H, J = 1.6, 8.8 Hz). HRMS (ESI+): Calcd. for [M+H]⁺ 397.0853, found 397.0840 (-1.3 mmu).

### Synthesis Example 1-5: Preparation of Q-sHMRG (Compound 6)

Compound 4 (36 mg, 0.091 mmol) obtained in Synthesis Example 1-4 was dissolved in dry DMF (2 mL). 3-Methyl-3-(2,4,5-trimethyl-3,6-dioxocyclohexa-1,4-dien-1-yl)butanoic acid (23 mg, 0.091 mmol), HATU (34 mg, 0.091 mmol), and DIEA (110 mL, 0.63 mmol) were added, and the mixture was stirred at 25°C for 2 hours. The solvent was concentrated under reduced pressure, and the residue was directly subjected to preparative purification. Preparative purification was performed by collecting a fraction having an absorption band with an absorption peak around 500 nm in MPLC (a gradient of A/B = 99/1 to 0/100 over 15 minutes; where A is 0.1% trifluoroacetic acid and B is methanol). By lyophilizing the obtained fraction, Compound 6 (13 mg, 34%) was obtained as an orange solid.

### Q-sHMRG (Compound 6)

¹H-NMR (DMSO-d6): δ 1.36 (s, 6H), 1.87 (s, 3H), 1.89 (s, 3H), 2.03 (s, 3H), 2.94 (s, 2H), 5.22 (s, 2H), 6.45 (d, 1H, J = 8.2 Hz), 6.6-7.8 (m, 7H), 8.95 (m, 1H). HRMS (ESI+): Calcd. for [M+H]⁺ 629.1958, found 629.1966 (+0.8 mmu).

### <Reference Example 1: Reactivity of Q-sHMRG with DT-diaphorase and Increase in Fluorescence>

Q-sHMRG prepared in Synthesis Example 1 was dissolved to give a final concentration of 1 µM in a HEPES buffer (100 mM, pH 7.4) containing CaCl₂ (1 mM), MgCl₂ (1 mM), DTT (dithiothreitol, 100 µM), and Triton X-100 (250 µM), and an absorption spectrum was measured. Then, NADH (100 µM) and NADH:quinone oxidoreductase (10 µg/mL, Toyobo, DAD-311) were added, and after incubation at 25°C for 4 hours, an absorption spectrum was measured again. Results are shown in (A) of FIG. 1.

Also, Q-sHMRG prepared in Synthesis Example 1 was dissolved to give a final concentration of 1 µM in a HEPES buffer (100 mM, pH 7.4) containing CaCl₂ (1 mM), MgCl₂ (1 mM), DTT (dithiothreitol, 100 µM), and Triton X-100 (250 µM), and then NADH at various concentrations and NADH:quinone oxidoreductase (10 µg/mL) were added. After incubation at 25°C for 30 minutes, fluorescence intensity was measured. Results are shown in (B) and (C) of FIG. 1.

According to (A) to (C) in FIG. 1, a fluorescence intensity of Q-sHMRG increased 200-fold or more (235-fold) by a reduction reaction by NADH:quinone oxidoreductase. Furthermore, according to (B) and (C) of FIG. 1, it was shown that a combination of Q-sHMRG and NADH:quinone oxidoreductase can detect NADH in as low a quantity as 0.01 µM, and it was revealed that reactivity of Q-sHMRG with NADH:quinone oxidoreductase is very high.

### <Example 1: Detection of Oxidative Activity of Lactate Dehydrogenase in Human Plasma>

Whether or not an activity of lactate dehydrogenase in human plasma can be detected (evaluated) by an evaluation method according to one embodiment of the present disclosure was examined.

To a HEPES buffer (100 mM, pH 7.4) containing CaCl₂ (1 mM), MgCl₂ (1 mM), DTT (dithiothreitol, 100 µM), and Triton X-100 (200 µM), Q-sHMRG (30 µM), Q-HMRG (30 µM) or resazurin (30 µM), lactate (1 mM), NAD⁺ (oxidized nicotinamide adenine dinucleotide, 500 µM), and NADH:quinone oxidoreductase (50 µg/mL, Toyobo, DAD-311) were added. Then, plasma obtained from a healthy human subject was added to give a 3000-fold dilution (preparation step, Step (A)). 40 µL of the solution (a pre-encapsulation aqueous solution) thus prepared was introduced into a microchamber array Simoa (registered trademark) disk (Quanterix). Subsequently, 80 µL of Fluorinert (registered trademark) FC-70 (Sigma-Aldrich) as a hydrophobic solvent was introduced into the microchamber array to remove an aqueous solution not introduced into wells, and thereby W/O droplets were formed in wells. After incubation at room temperature for 3 hours (reaction step, Step (B)), fluorescence images were acquired by an epifluorescence microscope Ti2 (Nikon) equipped with a 20× objective lens (Plan Apo 20×), an sCMOS camera (ORCA-Fusion C14440, Hamamatsu Photonics), a white light unit (X-Cite Xylis, Opto Science), and a motorized stage (fluorescence measurement step, Step (C)).

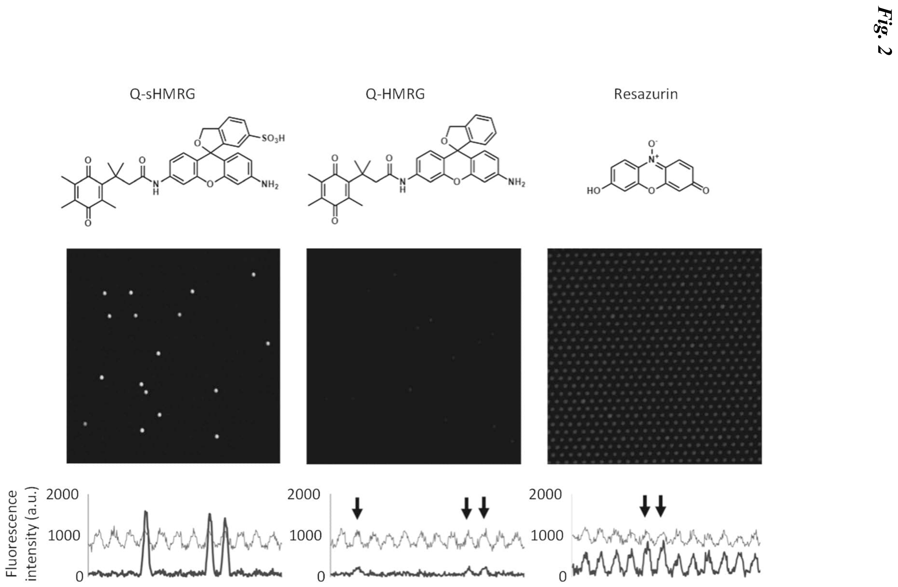

Results are shown in FIG. 2. In FIG. 2, arrows in line plots in results of Q-HMRG and resazurin indicate wells in which a fluorescence intensity derived from a fluorescent dye produced by metabolism of a fluorescent probe appears to have increased. According to FIG. 2, among wells of a microchamber array, in wells presumed to have been stochastically encapsulating lactate dehydrogenase, a fluorescence intensity derived from a fluorescent dye produced by metabolism of a fluorescent probe increased. Therefore, it was shown that, according to an evaluation method according to one embodiment of the present disclosure, an oxidative activity of a single molecule of a dehydrogenase can be evaluated at the single-molecule level. Furthermore, when comparing results among fluorescent dyes, in a case of using Q-sHMRG having a sulfo group and high water solubility, a ratio (S/N ratio) of a fluorescence intensity of a well encapsulating lactate dehydrogenase to a fluorescence intensity of a well not encapsulating lactate dehydrogenase was greater. Also, even when compared with a case where the same experiment was performed using SiRWST-3 (Non Patent Literature 5) or Q-dsAMC (Non Patent Literature 2) as a fluorescent probe (provided that, in a case of using SiRWST-3, 100 µM of 1-methoxy-5-methylphenazinium methyl sulfate (1-MeO-PMS), which is an electron mediator, was added in place of an NADH:quinone oxidoreductase), a case of using Q-sHMRG, Q-HMRG, or resazurin had a larger S/N ratio, and, among them, in a case of using Q-sHMRG, a ratio (S/N ratio) of a fluorescence intensity of a well encapsulating lactate dehydrogenase to a fluorescence intensity of a well not encapsulating lactate dehydrogenase was greater.

### <Example 2: Detection of Oxidative Activities of Various Dehydrogenases in Human Plasma>

Whether or not activities of dehydrogenases corresponding to respective substrates can be detected (evaluated) by using substrates of various dehydrogenases in an evaluation method according to one embodiment of the present disclosure was examined.

To a HEPES buffer (100 mM, pH 7.4) containing CaCl₂ (1 mM), MgCl₂ (1 mM), DTT (dithiothreitol, 100 µM), and Triton X-100 (200 µM), Q-sHMRG (30 µM), a substrate of a dehydrogenase (1 mM), NAD⁺ (100 µM), and NADH:quinone oxidoreductase (50 µg/mL) were added. Then, plasma obtained from a healthy human subject was added to give a 1000-fold dilution (preparation step, Step (A)). 40 µL of the solution (a pre-encapsulation aqueous solution) thus prepared was introduced into a microchamber array Simoa (registered trademark) disk (Quanterix). Subsequently, 80 µL of Fluorinert (registered trademark) FC-70 (Sigma-Aldrich) as a hydrophobic solvent was introduced into the microchamber array to remove an aqueous solution not introduced into wells, and thereby W/O droplets were formed in wells. After incubation at room temperature for 3 hours (reaction step, Step (B)), fluorescence images were acquired by an epifluorescence microscope Ti2 (Nikon) with the same setup as in Example 1 (fluorescence measurement step, Step (C)).

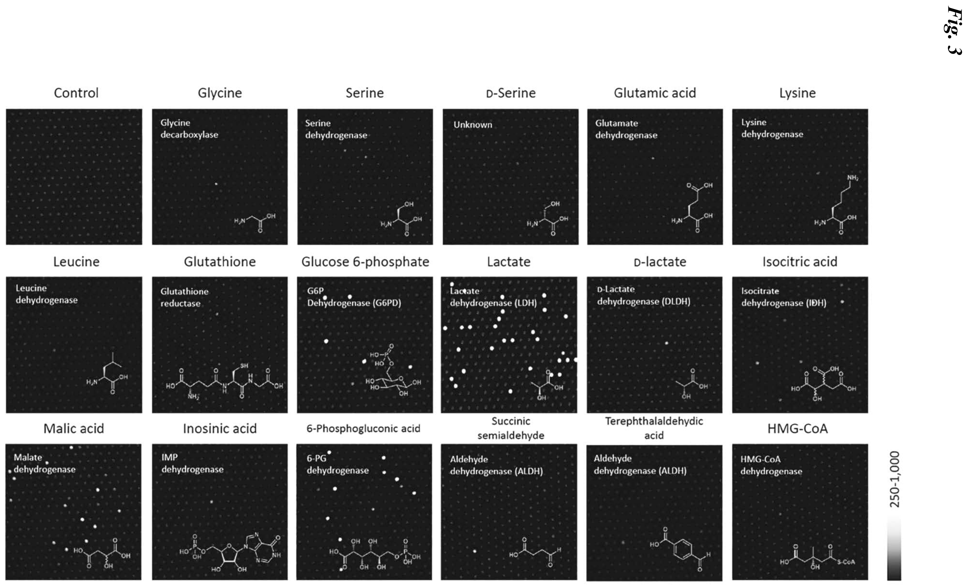

Evaluation was performed in cases of using glycine, serine, D-serine, glutamic acid, lysine, leucine, glutathione, glucose-6-phosphate, lactate, D-lactate, isocitrate, malate, inosinic acid, 6-phosphogluconate, succinic semialdehyde, terephthalaldehydic acid, or HMG-CoA (hydroxymethylglutaryl CoA) as substrates of a dehydrogenase. Dehydrogenases corresponding to these substrates are as shown in FIG. 3.

Results are shown in FIG. 3. According to FIG. 3, only by changing a type of a substrate of a dehydrogenase to be added, a proportion of wells in which fluorescence was observed and fluorescence intensities in fluorescence images obtained varied in various ways. Thus, it was revealed that, by an evaluation method according to one embodiment of the present disclosure, even when only one kind of fluorescent probe is used, by changing a type of a substrate of a dehydrogenase to be added, activities of multiple types of dehydrogenases can be detected (evaluated) at the single-molecule level.

### <Example 3: Detection of an oxidative activity of a dehydrogenase when various nucleoside diphosphate derivatives were used>

It was investigated whether, when various nucleoside diphosphate derivatives are used as coenzymes, oxidative activities of different subtypes of a dehydrogenase that target the same substrate can be detected (evaluated) by an evaluation method according to one embodiment of the present disclosure.

To a HEPES buffer (100 mM, pH 7.4) containing CaCl₂ (1 mM), MgCl₂ (1 mM), DTT (dithiothreitol, 100 µM), and Triton X-100 (200 µM), Q-sHMRG (30 µM), a substrate of a dehydrogenase (1 mM), a nucleoside diphosphate derivative (500 µM), and NADH:quinone oxidoreductase (50 µg/mL) were added. Thereafter, plasma obtained from a healthy human subject was added to give a 1000-fold dilution (a preparation step, Step (A)). 40 µL of the solution (a pre-encapsulation aqueous solution) prepared was introduced into a microchamber array, Simoa (registered trademark) disk (Quanterix). Subsequently, 80 µL of Fluorinert (registered trademark) FC-70 (Sigma-Aldrich) as a hydrophobic solvent was introduced into the microchamber array to remove a solution not introduced into wells, and thereby W/O droplets were formed in wells. After incubation at room temperature for 3 hours (a reaction step, Step (B)), fluorescence images were acquired by an epifluorescence microscope Ti2 (Nikon) with the same setup as in Example 1 (a fluorescence measurement step, Step (C)).

As substrates of a dehydrogenase, lactate, glucose-6-phosphate, isocitrate, or 6-phosphogluconate was used. As nucleoside diphosphate derivatives, NAD⁺ (Oxidized nicotinamide adenine dinucleotide), NADP⁺ (Oxidized nicotinamide adenine dinucleotide phosphate), Thio-NAD⁺ (Oxidized thionicotinamide adenine dinucleotide), or NGD⁺ (Oxidized nicotinamide guanine dinucleotide) shown in the following chemical formula was used.

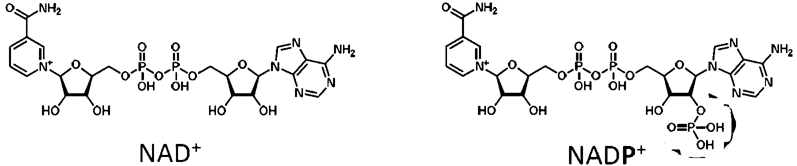

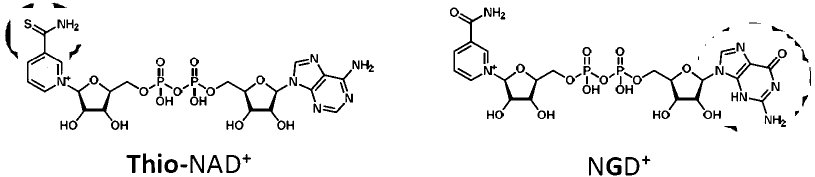

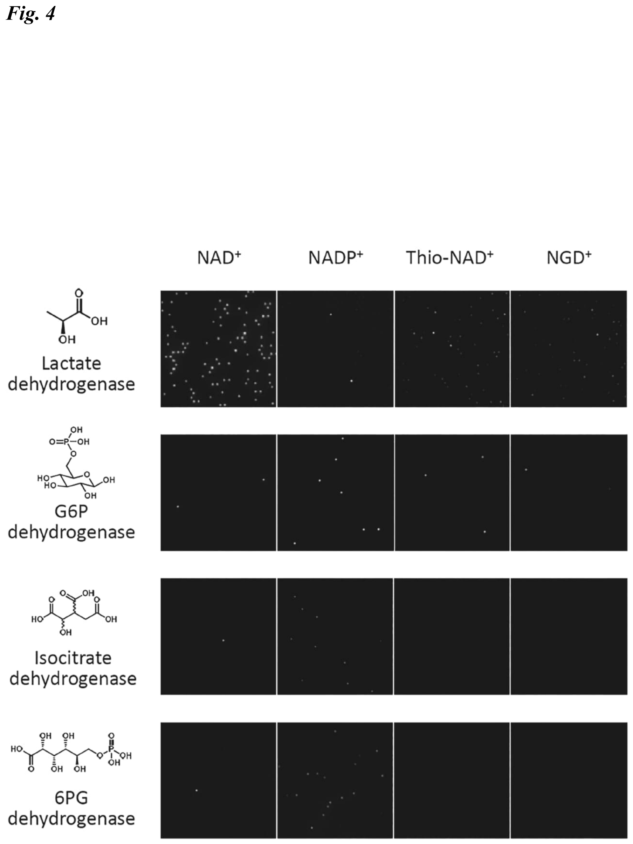

Results are shown in FIG. 4. According to FIG. 4, depending on a type of an added nucleoside diphosphate derivative, even when the same substrate of a dehydrogenase was used, a pattern of fluorescence images (a proportion of wells in which strong fluorescence was observed and fluorescence intensity) observed greatly differed. This is presumed to be because, depending on a subtype of a dehydrogenase present in each well, a type of a nucleoside diphosphate derivative that can be used as a coenzyme and an efficiency of its use greatly differ. Therefore, by the evaluation method according to one embodiment of the present disclosure, even when only one kind of fluorescent probe is used, it was clearly demonstrated that oxidative activities of different subtypes of a dehydrogenase that target the same substrate can be detected (evaluated) at the single-molecule level of a dehydrogenase by changing a type of an added nucleoside diphosphate derivative. By such an evaluation method according to one embodiment of the present disclosure, it is possible to detect (evaluate) at the single-molecule level of an enzyme an oxidative activity of a dehydrogenase of a minor subtype that can use a specific nucleoside diphosphate derivative with high efficiency, which would be buried in a bulk evaluation system.

### <Example 4: Dependence of fluorescence images on a substrate of a dehydrogenase and a nucleoside diphosphate derivative when plasma derived from healthy subjects and from patients with pancreatic cancer was used>

It was investigated whether differences in patterns of fluorescence images were observed in plasma derived from healthy subjects and from patients with pancreatic cancer by using the evaluation method according to one embodiment of the present disclosure demonstrated in Examples 1 to 3. In particular, it was investigated whether differences in patterns of fluorescence images arise in plasma derived from healthy subjects and from patients with pancreatic cancer depending on a type of a substrate of a dehydrogenase and a type of a nucleoside diphosphate derivative.

To a HEPES buffer (100 mM, pH 7.4) containing CaCl₂ (1 mM), MgCl₂ (1 mM), DTT (dithiothreitol, 100 µM), and Triton X-100 (200 µM), Q-sHMRG (30 µM), a substrate of a dehydrogenase (1 mM), a nucleoside diphosphate derivative (500 µM), and NADH:quinone oxidoreductase (50 µg/mL) were added. Thereafter, plasma (a sample) obtained from a healthy human subject or from a human subject suffering from pancreatic cancer was added to give a 1000-fold dilution (a preparation step, Step (A)). 40 µL of the solution (a pre-encapsulation aqueous solution) prepared was introduced into a microchamber array, Simoa (registered trademark) disk (Quanterix). Subsequently, 80 µL of Fluorinert (registered trademark) FC-70 (Sigma-Aldrich) as a hydrophobic solvent was introduced into the microchamber array to remove a solution not introduced into wells, and W/O droplets were formed in wells. After incubation at room temperature for 3 hours (a reaction step, Step (B)), fluorescence images were acquired by an epifluorescence microscope Ti2 (Nikon) with the same setup as in Example 1 (a fluorescence measurement step, Step (C)).

As substrates of a dehydrogenase, glucose-6-phosphate or 6-phosphogluconate was used. As nucleoside diphosphate derivatives, NAD⁺, NADP⁺, or Thio-NAD⁺ was used.

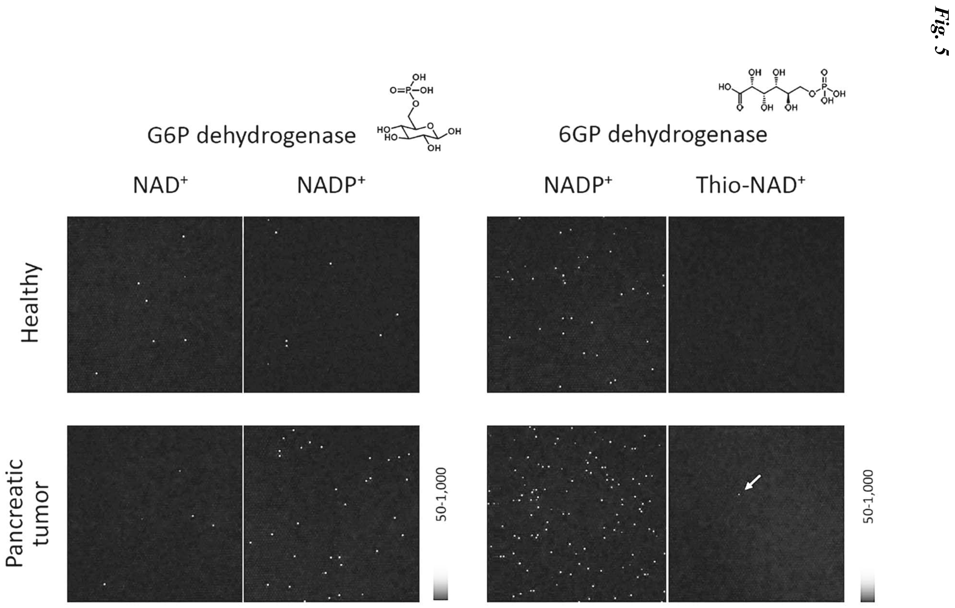

Results are shown in FIG. 5. According to FIG. 5, even when the same fluorescent probe was used differences in patterns of fluorescence images in plasma derived from healthy subjects and from patients with pancreatic cancer greatly differed depending on a type of a substrate of a dehydrogenase and a type of a nucleoside diphosphate derivative. For example, when 6-phosphogluconate was used as a substrate of a dehydrogenase and Thio-NAD⁺ was used as a nucleoside diphosphate derivative, respectively, bright spots were not observed in plasma derived from healthy subjects, whereas bright spots were observed in plasma derived from patients with pancreatic cancer, and differences in patterns of fluorescence images between the two could be clearly evaluated. Thus, it was shown that, by the evaluation method according to one embodiment of the present disclosure, it is possible to discover an evaluation system in which patterns of fluorescence images tend to arise among multiple groups to be discriminated, independently of a type of a fluorescent probe, by changing a type of a substrate of a dehydrogenase and a type of a nucleoside diphosphate derivative.

### <Example 5: Detection of activities of metabolic enzymes contained in plasma derived from patients with pancreatic cancer>

To a HEPES buffer (100 mM, pH 7.4) containing CaCl₂ (1 mM), MgCl₂ (1 mM), DTT (dithiothreitol, 100 µM), and Triton X-100 (200 µM), Q-sHMRG (30 µM), glucose (1 mM), ATP (1 mM), NAD⁺ (500 µM), NADP⁺ (500 µM), glucose-6-phosphate dehydrogenase (G6PDH, 1 µg/mL, manufactured by R&D Systems), and NADH:quinone oxidoreductase (5 µg/mL) were added. Thereafter, plasma (a sample) obtained from a human subject suffering from pancreatic cancer was added to give a 1000-fold dilution (a preparation step, Step (A)). 40 µL of the solution (a pre-encapsulation aqueous solution) prepared was introduced into a microchamber array, Simoa (registered trademark) disk (Quanterix). Subsequently, 80 µL of Fluorinert (registered trademark) FC-70 (Sigma-Aldrich) as a hydrophobic solvent was introduced into the microchamber array to remove a solution not introduced into wells, and thereby W/O droplets were formed in wells. After incubation at room temperature for 3 hours (a reaction step, Step (B)), fluorescence images were acquired by an epifluorescence microscope Ti2 (Nikon) with the same setup as in Example 1 (a fluorescence measurement step, Step (C)). Also, as a control experiment, the same test was performed under a condition in which G6PDH was not added (G6PDH (-)).

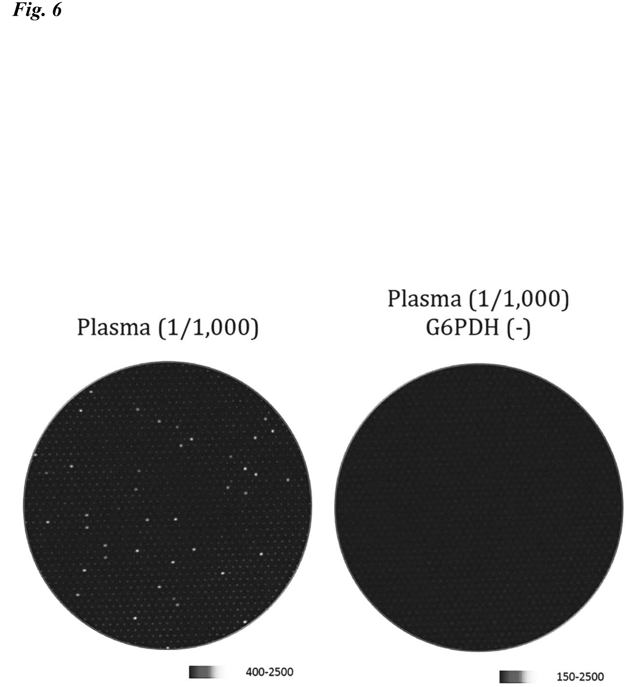

Results are shown in FIG. 6. According to FIG. 6, under a condition in which G6PDH was added, fluorescence derived from sHMRG was observed because all of a metabolic reaction by hexokinase, an oxidation reaction by G6PDH, and a reduction reaction by the DT-diaphorase in a coupled assay system occurred, whereas under a condition in which G6PDH was not added, fluorescence was not observed. Thus, it was clearly demonstrated that a metabolic activity of a metabolic enzyme can be detected (evaluated) at the single-molecule level of a metabolic enzyme by a method according to one embodiment of a third aspect of the present disclosure.

### <Example 6: Detection of activities of dehydrogenases contained in plasma derived from acute liver injury model mice>

In accordance with a method described in a literature (Tatsuya Ukegawa et al., "Thioester-Based Coupled Fluorogenic Assays in Microdevice for the Detection of Single-Molecule Enzyme Activities of Esterases with Specified Substrate Recognition", Adv. Sci. 11, 2306559 (2024)), mice treated with thioacetamide (TAA) were produced as acute liver injury model mice. Blood was collected from the produced acute liver injury model mice, and plasma (a sample) was collected from the blood. Detection of activities of dehydrogenases was performed in accordance with the same method as in Example 2 using the plasma. As substrates of a dehydrogenase, glucose-6-phosphate and lactate were used.

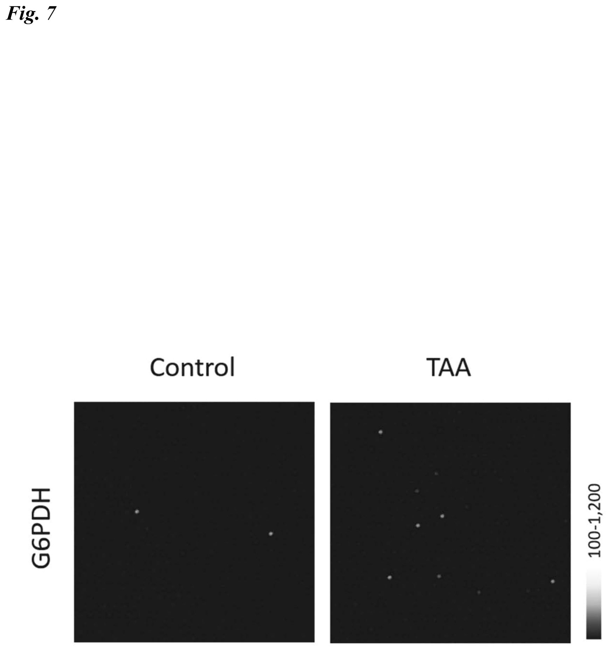

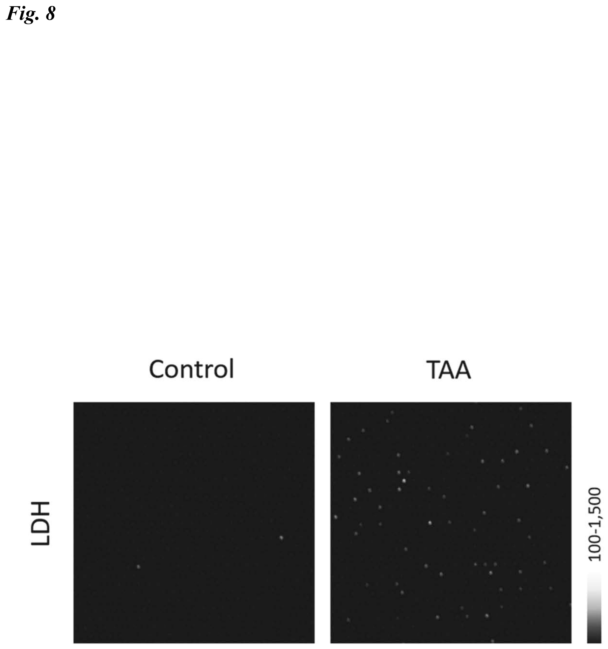

FIG. 7 is a diagram showing fluorescence images obtained, under a condition in which glucose-6-phosphate was added, when using samples derived from mice that were not subjected to TAA treatment (control group) or mice that were subjected to TAA treatment (TAA group). FIG. 8 is a diagram showing fluorescence images obtained, under a condition in which lactate was added, when using samples derived from mice that were not subjected to TAA treatment (control group) or mice that were subjected to TAA treatment (TAA group). According to FIG. 7 and FIG. 8, when samples derived from acute liver injury model mice were used, the number of wells in which fluorescence was detected was larger compared with when samples derived from control mice were used. From the results, it was clearly demonstrated that, in samples derived from acute liver injury model mice, activities of G6PDH, which is a dehydrogenase that uses glucose-6-phosphate as a substrate, and lactate dehydrogenase (LDH), which is a dehydrogenase that uses lactate as a substrate, are enhanced, and that the enhancement can be detected (evaluated) by a method according to one embodiment of a first aspect of the present disclosure.

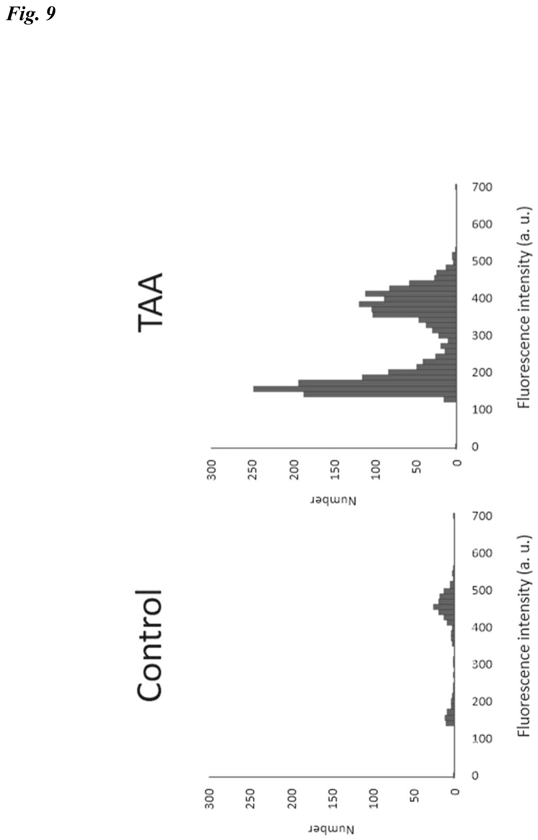

FIG. 9 is a histogram of fluorescence images acquired under the same conditions as in FIG. 7, showing distributions of fluorescence intensity for each well in which fluorescence was detected. According to FIG. 9, interestingly, samples derived from acute liver injury model mice had two populations of activity of G6PDH, and the number of wells in which G6PDH having activity in both populations was encapsulated increased in samples derived from acute liver injury model mice. However, rates of increase of the two differed, and the number of wells in which G6PDH having activity in a low-activity population was encapsulated increased more. From these results, it was clearly demonstrated that, by a method according to one embodiment of a first aspect of the present disclosure, it is possible to detectively distinguish multiple populations that have the same metabolic activity and have different levels of the activity, and it is also possible to detect even a number of a low-activity population, which may be difficult to detect in a bulk system containing many enzyme molecules.

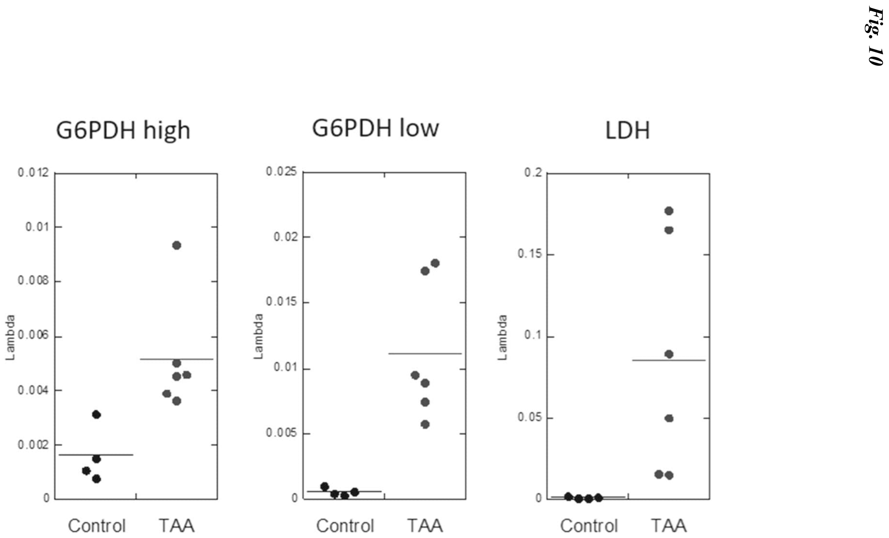

FIG. 10 is a diagram, of fluorescence images acquired under the same conditions as in FIG. 7 and FIG. 8, showing a proportion of wells in which fluorescence was detected (lambda). From results in FIG. 10, it was confirmed that, in any of a high-activity population of G6PDH (G6PDH high), a low-activity population of G6PDH (G6PDH low), and LDH, the number of wells in which fluorescence was detected was larger in acute liver injury model mice. Therefore, by a method according to one embodiment of a first aspect of the present disclosure in which glucose-6-phosphate or lactate is used as a substrate, it was suggested that diagnosis of a disease such as acute liver injury, assistance of diagnosis, and acquisition of data for diagnosis can be achieved.

### <Example 7: Detection of activities of dehydrogenases contained in cerebrospinal fluid derived from patients with primary central nervous system malignant lymphoma (PCNSL)>

It was investigated whether activities of dehydrogenases contained in cerebrospinal fluid (CSF) derived from patients with primary central nervous system malignant lymphoma (PCNSL) can be detected by a method according to one embodiment of a first aspect of the present disclosure. As CSF samples, cerebrospinal fluid collected from patients whose morbidity with PCNSL was histologically determined and who received surgery and chemotherapy based on high-dose administration of methotrexate for the treatment at the Neuro-Oncology/Neurosurgery Department of Saitama Medical University International Medical Center between March 2019 and December 2023 was used. This study was approved by the Institutional Review Board of Saitama Medical University International Medical Center (approval number: 2021-017) and the University of Tokyo (approval number: 5-2). Written informed consent was obtained from all patients. The study was conducted in accordance with the Declaration of Helsinki. From newly diagnosed PCNSL patients, CSF was collected before treatment, and CSF samples were also sequentially collected during treatment and at the end of chemotherapy. In recurrent PCNSL, CSF was collected when recurrence in the central nervous system was observed by MRI with a contrast agent. All patients were immunocompetent and HIV-negative. All CSF was collected by lumbar puncture. For comparison, CSF was also collected from patients with glioblastoma or metastatic brain tumor. A pathological diagnosis was made by a senior neuropathologist according to the 2016 World Health Organization (WHO) classification system. CSF was centrifuged at 500×g for 10 minutes to remove cells, and a supernatant was stored at -80°C within 30 minutes after collection. Using the CSF samples thus obtained, detection of activities of dehydrogenases was performed according to the same method as in Example 2. As a substrate of a dehydrogenase, glucose-6-phosphate was used.

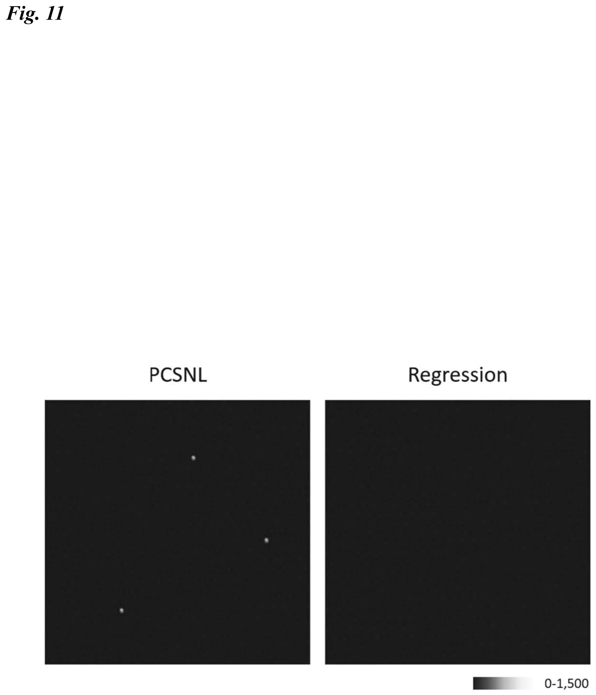

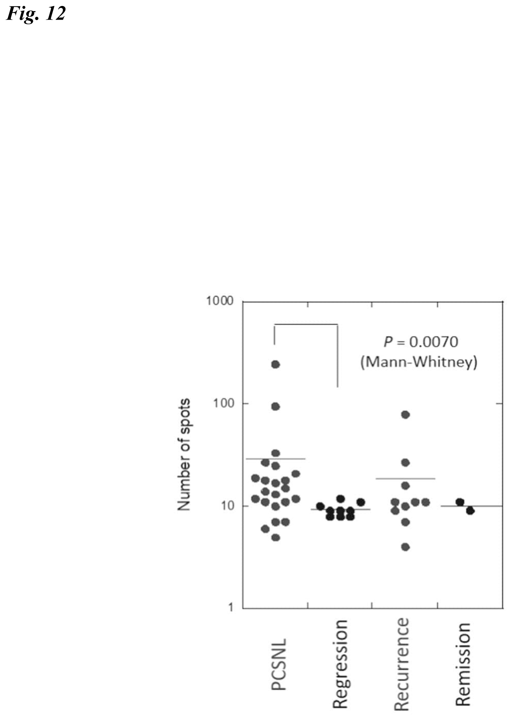

FIG. 11 is a diagram showing fluorescence images obtained when CSF samples derived from PCNSL patients (PCNSL group) and subjects in whom tumors regressed (Regression group) were used. FIG. 12 is a diagram showing results of counting numbers of wells in which fluorescence was observed in fluorescence images obtained under the same conditions as in FIG. 11 when CSF samples derived from PCNSL patients (PCNSL group), subjects in whom tumors regressed (Regression group), patients in whom PCNSL recurred (Recurrence group), and subjects in whom PCNSL remitted (Remission group) were used. As shown in FIG. 11 and FIG. 12, in CSF samples of the PCNSL group and the Recurrence group, in which symptoms of PCNSL were evident, the numbers of wells in which fluorescence was observed were larger than those in CSF samples of the Regression group and the Remission group, in which symptoms are in recovery trend. In particular, the number of wells in which fluorescence was observed in the PCNSL group was significantly larger than that in the Regression group (P value in a Mann-Whitney test was 0.0070). From these results, it was clearly demonstrated that, in CSF samples of PCNSL patients, an activity of G6PDH, which is a dehydrogenase that uses glucose-6-phosphate as a substrate, is enhanced, and that the enhancement can be detected (evaluated) by a method according to one embodiment of a first aspect of the present disclosure. In addition, it was shown that activities of dehydrogenases in cerebrospinal fluid can be detected by a method according to one embodiment of a first aspect of the present disclosure. Thereby, it was also clearly demonstrated that applicable biological samples of a method according to one embodiment of a first aspect of the present disclosure is not limited to blood samples. In addition, it was suggested that diagnosis of a disease such as PCNSL, assistance of diagnosis, and acquisition of data for diagnosis can be achieved by a method according to one embodiment of a first aspect of the present disclosure in which glucose-6-phosphate is used as a substrate.

### <Comparative Example 1: Detection of dehydrogenase activities using a bulk measurement system>

Detection of dehydrogenase activities using a bulk measurement system was carried out, and by comparing with results of Example 2, superiority of performing a method according to one embodiment of a first aspect of the present disclosure at the single-molecule level of a dehydrogenase was investigated. To a HEPES buffer (100 mM, pH 7.4) containing CaCl₂ (1 mM), MgCl₂ (1 mM), DTT (dithiothreitol, 100 µM), and Triton X-100 (200 µM), Q-sHMRG (10 µM), a substrate of a dehydrogenase (1 mM), NAD⁺ (500 µM), NADP⁺ (500 µM), and NADH:quinone oxidoreductase (50 µg/mL) were added. The solution was loaded into each well of a 384-well microplate (manufactured by Greiner). Thereafter, the same plasma sample as that in Example 2 was added to give 1000-fold or 100-fold dilution. Using a microplate reader (2103 EnVision, PerkinElmer), rates of increase in fluorescence in each well were measured for 30 minutes at room temperature.

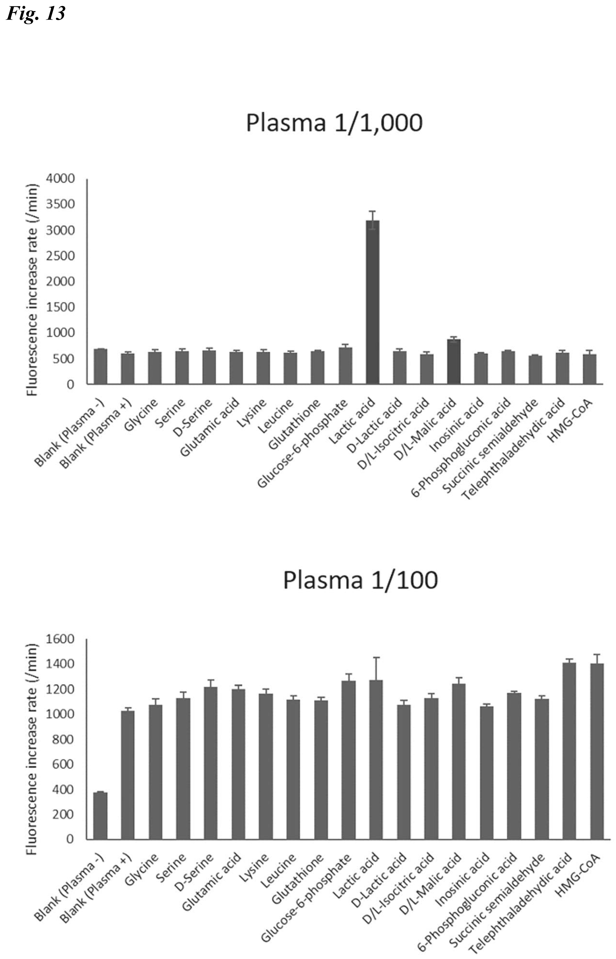

FIG. 13 is a diagram showing rates of increase in fluorescence (/min) in the presence of substrates of various metabolic enzymes when a plasma sample was added to give 1000-fold or 100-fold dilution. Results in FIG. 13 are shown as mean ± standard deviation (Mean ± S.D.) for n = 3, wherein Blank (plasma-) shows a control in which neither a plasma sample nor a substrate was added, and Blank (plasma+) shows a control in which a substrate was not added. According to FIG. 13, when an evaluation using the same enzymatic reactions as in one embodiment of a first aspect of the present disclosure was carried out using a bulk system, not a single molecule evaluation system, activities of dehydrogenases could be detected only when lactate and malate were used as substrates under a 1000-fold dilution condition at best. This is opposite to that, as demonstrated in Example 2, when a method of one embodiment of a first aspect of the present disclosure was implemented as a single molecule evaluation system, activities of dehydrogenases could be detected when many kinds of substrates were used. From these results, it was demonstrated that a method of one embodiment of a first aspect of the present disclosure enables detection of a wide range of dehydrogenases whose signals would be buried in background signals and would be difficult to detect in a bulk system, by using a system containing a single molecule of a dehydrogenase.

### <Synthesis Example 2: Azo-rhodamine>

Azo-rhodamine was designed and synthesized as a fluorescent probe whose fluorescence intensity increases by being metabolized by an azoreductase, which is a kind of DT-diaphorase. Azo-rhodamine is a fluorescent probe whose fluorescence intensity increases by metabolism by an azoreductase shown in the following reaction scheme. 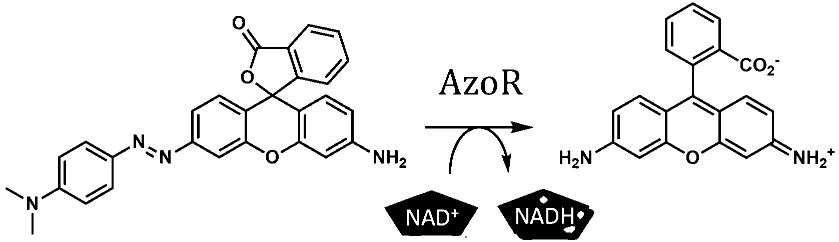 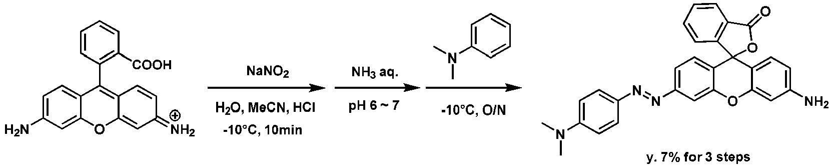 A known fluorescent dye, rhodamine 110, 20 mg (0.055 mmol), which is also available from suppliers, was dissolved in H₂O/MeCN/Conc. HCl (4/1/1 mL) and stirred in an ice bath under an argon atmosphere. NaNO₂ 3.65 mg (0.055 mmol) was dissolved in 183 µL of H₂O, and the solution was added dropwise, followed by stirring for 2 hours. A pH was adjusted to 6-7 by adding aqueous ammonia, and 13.8 µL (0.11 mmol) of N,N-dimethylaniline dissolved in AcCN was added. The reaction mixture was purified by preparative MPLC (a gradient of A/B = 95/5 to 0/100 over 15 minutes; wherein A is a 0.1 M triethylamine acetate buffer, and B is AcCN containing 0.1 M triethylamine acetate). A fraction having an absorption band with a peak around 500 nm in absorbance was collected and lyophilized to afford a compound, 1.7 mg (6.7%).

### Azo-rhodamine

¹H-NMR (400 MHz, CD₃OD): δ 8.36 (d, J = 7.8 Hz, 1H), 8.05 (d, J = 1.4 Hz, 1H), 7.94-7.82 (m, 5H), 7.47 (d, J = 7.3 Hz, 1H), 7.32 (d, J = 8.7 Hz, 1H), 7.20 (d, J = 9.1 Hz, 1H), 6.98-6.93 (m, 2H), 6.88 (dd, J = 7.3, 2.3 Hz, 2H), 3.17 (s, 6H)

### <Example 8: Detection of activities of dehydrogenases using an azoreductase as a DT-diaphorase>

To a HEPES buffer (100 mM, pH 7.4) containing CaCl₂ (1 mM), MgCl₂ (1 mM), DTT (100 µM), and Triton X-100 (250 µM), Azo-rhodamine (30 µM), lactate (1 mM), NAD⁺ (500 µM), flavin mononucleotide (FMN, 0.01 µM), and azoreductase (5 µg/mL, a recombinant enzyme obtained by a method described in Non Patent Literature 3) were added. Thereafter, plasma obtained from a healthy human subject was added to give a 3000-fold dilution (a preparation step, Step (A)). 40 µL of the solution (a pre-encapsulation aqueous solution) prepared was introduced into a microchamber array, Simoa (registered trademark) disk (Quanterix). Subsequently, 80 µL of Fluorinert (registered trademark) FC-70 (Sigma-Aldrich) as a hydrophobic solvent was introduced into the microchamber array to remove a solution not introduced into wells, and thereby W/O droplets were formed in wells. After incubation at room temperature for 20 hours (a reaction step, Step (B)), fluorescence images were acquired by an epifluorescence microscope Ti2 (Nikon) with the same setup as in Example 1 (a fluorescence measurement step, Step (C)).

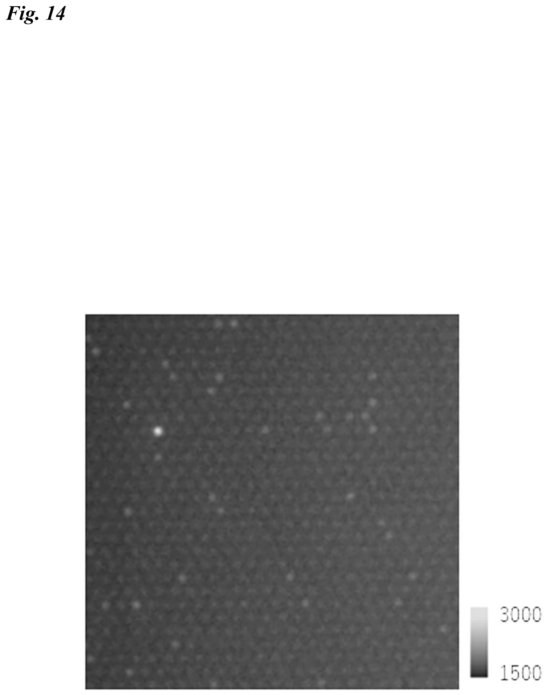

FIG. 14 is a diagram showing a fluorescence image of a device 20 hours after addition of plasma. According to FIG. 14, fluorescence was detected from wells in which LDH was considered to be stochastically encapsulated. Thus, it was clearly demonstrated that a method of one embodiment of a first aspect of the present disclosure can evaluate (detect) activities of dehydrogenases even when azoreductase is used as a DT-diaphorase. Thereby, it was shown that a DT-diaphorase according to a method of one embodiment of a first aspect of the present disclosure is not limited to NADH:quinone oxidoreductase.

## Claims

1. A method for evaluating an oxidative activity of a dehydrogenase, comprising the following steps (A) to (C):
Step (A): a step of preparing an aqueous solution containing a single molecule of the dehydrogenase, a substrate of the dehydrogenase, a nucleoside diphosphate derivative, a DT-diaphorase, and a fluorescent probe;
Step (B): a step of causing the dehydrogenase and the DT-diaphorase to catalyze a redox reaction in the aqueous solution prepared in the step (A); and
Step (C): a step of measuring fluorescence of the aqueous solution resulting from the step (B);
wherein the dehydrogenase is capable of using an oxidized form of the nucleoside diphosphate derivative as a coenzyme,
wherein the DT-diaphorase is capable of using a reduced form of the nucleoside diphosphate derivative as a coenzyme,
and wherein the fluorescent probe is a compound whose fluorescence intensity increases upon reduction by the DT-diaphorase.

2. The evaluation method according to claim 1, wherein the step (A) is a step of filling an aqueous solution containing the dehydrogenase, the substrate of the dehydrogenase, the nucleoside diphosphate derivative, the DT-diaphorase, and the fluorescent probe into a plurality of accommodating portions of a microchamber array having a hydrophobic surface, the step comprising the following steps (i) and (ii) in this order:
(i) from an opening of each of the accommodating portions, flowing the aqueous solution containing the dehydrogenase, the substrate of the dehydrogenase, the nucleoside diphosphate derivative, the DT-diaphorase, and the fluorescent probe into each of the plurality of accommodating portions so that a number-average number of molecules of the dehydrogenase to be filled in each of the plurality of accommodating portions is 0.0002 to 0.5; and
(ii) closing the opening with a hydrophobic solvent.

3. The evaluation method according to claim 1 or 2, wherein the fluorescent probe has at least one substituent selected from the group consisting of - CO₂H, -PO₃H₂, and -SO₃H.

4. The evaluation method according to claim 1 or 2, wherein the nucleoside diphosphate derivative in an oxidized form is at least one compound selected from the group consisting of compounds represented by the following formula (I) or (II): 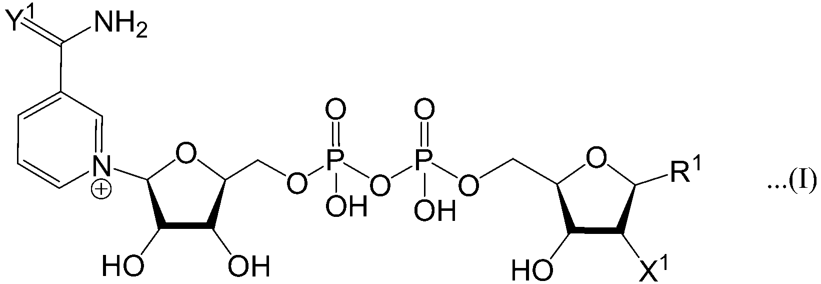 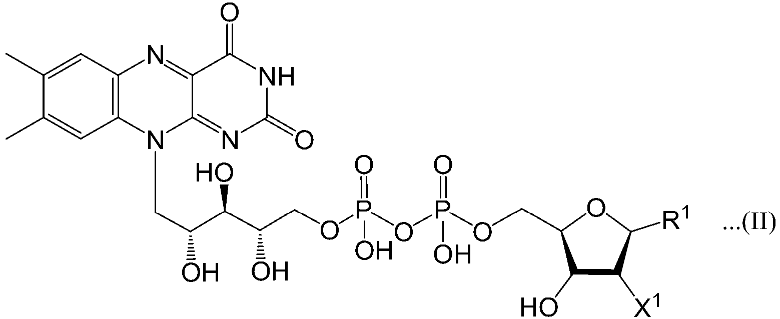 , wherein R¹ is a monovalent group, X¹ is -H, -OH, or -PO₃H₂, and Y₁ is O or S.

5. The evaluation method according to claim 1 or 2, wherein the DT-diaphorase is an NADH:quinone oxidoreductase, an azoreductase, or a nitroreductase.

6. The evaluation method according to claim 1 or 2, wherein the dehydrogenase is a dehydrogenase contained in at least one selected from the group consisting of plasma, serum, urine, saliva, tears, cerebrospinal fluid, tissue homogenate, cell homogenate, and extracts thereof.

7. A method for evaluating an oxidative activity of a dehydrogenase, comprising the following steps (A-1) to (C-1) and steps (A-2) to (C-2):
Step (A-1): a step of preparing an aqueous solution containing a single molecule of the dehydrogenase, a substrate of the dehydrogenase, a first nucleoside diphosphate derivative, a DT-diaphorase, and a fluorescent probe;
Step (B-1): a step of causing the dehydrogenase and the DT-diaphorase to catalyze a redox reaction in the aqueous solution prepared in the step (A-1);
Step (C-1): a step of measuring fluorescence of the aqueous solution resulting from the step (B-1);
Step (A-2): a step of preparing an aqueous solution containing a single molecule of the dehydrogenase, the substrate of the dehydrogenase, a second nucleoside diphosphate derivative, the DT-diaphorase, and the fluorescent probe;
Step (B-2): a step of causing the dehydrogenase and the DT-diaphorase to catalyze a redox reaction in the aqueous solution prepared in the step (A-2); and
Step (C-2): a step of measuring fluorescence of the aqueous solution resulting from the step (B-2);
wherein the first nucleoside diphosphate derivative is different from the second nucleoside diphosphate derivative,
wherein the dehydrogenase is capable of using an oxidized form of the first nucleoside diphosphate derivative and an oxidized form of the second nucleoside diphosphate derivative as a coenzyme,
wherein the DT-diaphorase is capable of using a reduced form of the first nucleoside diphosphate derivative and a reduced form of the second nucleoside diphosphate derivative as a coenzyme,
and wherein the fluorescent probe is a compound whose fluorescence intensity increases upon reduction by the DT-diaphorase.

8. A method for evaluating an oxidative activity of a dehydrogenase, comprising the following steps (A-3) to (C-3) and steps (A-4) to (C-4):
Step (A-3): a step of preparing an aqueous solution containing a single molecule of a first dehydrogenase, a substrate of the first dehydrogenase, a nucleoside diphosphate derivative, a DT-diaphorase, and a fluorescent probe;
Step (B-3): a step of causing the first dehydrogenase and the DT-diaphorase to catalyze a redox reaction in the aqueous solution prepared in the step (A-3);
Step (C-3): a step of measuring fluorescence of the aqueous solution resulting from the step (B-3);
Step (A-4): a step of preparing an aqueous solution containing a single molecule of a second dehydrogenase, a substrate of the second dehydrogenase, the nucleoside diphosphate derivative, the DT-diaphorase, and the fluorescent probe;
Step (B-4): a step of causing the second dehydrogenase and the DT-diaphorase to catalyze a redox reaction in the aqueous solution prepared in the step (A-4); and
Step (C-4): a step of measuring fluorescence of the aqueous solution resulting from the step (B-4);
wherein the substrate of the first dehydrogenase is different from the substrate of the second dehydrogenase,
wherein the first dehydrogenase and the second dehydrogenase are capable of using an oxidized form of the nucleoside diphosphate derivative as a coenzyme,
wherein the DT-diaphorase is capable of using a reduced form of the nucleoside diphosphate derivative as a coenzyme,
and wherein the fluorescent probe is a compound whose fluorescence intensity increases upon reduction by the DT-diaphorase.

9. A kit for evaluating an oxidative activity of a dehydrogenase, comprising a substrate of the dehydrogenase, a nucleoside diphosphate derivative, a DT-diaphorase, and a fluorescent probe,
wherein the dehydrogenase is capable of using an oxidized form of the nucleoside diphosphate derivative as a coenzyme,
wherein the DT-diaphorase is capable of using a reduced form of the nucleoside diphosphate derivative as a coenzyme,
and wherein the fluorescent probe is a compound whose fluorescence intensity increases upon reduction by the DT-diaphorase.

10. A method for evaluating a metabolic activity of a metabolic enzyme, comprising the following steps (A) to (C):
Step (A): a step of preparing an aqueous solution containing a single molecule of the metabolic enzyme, a substrate of the metabolic enzyme, a dehydrogenase, a nucleoside diphosphate derivative, a DT-diaphorase, and a fluorescent probe;
Step (B): a step of causing the metabolic enzyme, the dehydrogenase, and the DT-diaphorase to catalyze an enzymatic reaction in the aqueous solution prepared in the step (A); and
Step (C): a step of measuring fluorescence of the aqueous solution resulting from the step (B);
wherein the dehydrogenase is a dehydrogenase that uses, as a substrate, a metabolite resulting from the metabolic enzyme metabolizing the substrate of the metabolic enzyme,
wherein the dehydrogenase is capable of using an oxidized form of the nucleoside diphosphate derivative as a coenzyme,
wherein the DT-diaphorase is capable of using a reduced form of the nucleoside diphosphate derivative as a coenzyme,
and wherein the fluorescent probe is a compound whose fluorescence intensity increases upon reduction by the DT-diaphorase.
